(19)

**Europäisches Patentamt**
**European Patent Office**
**Office européen des brevets**

(11) **EP 4 722 339 A2**

(12) **EUROPEAN PATENT APPLICATION**

(43) Date of publication:
08.04.2026 Bulletin 2026/15

(21) Application number: 25217055.0

(22) Date of filing: 20.03.2020

(51) International Patent Classification (IPC):
**C12N 5/00** (2006.01)

(52) Cooperative Patent Classification (CPC):
(C-Sets available)
**A61K 45/06; A61K 31/675; A61K 31/7076;**
**A61K 40/11; A61K 40/42; A61P 35/02;**
**C12N 5/0638;** A61K 2239/31; A61K 2239/38;
A61K 2239/48 (Cont.)

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(62) Document number(s) of the earlier application(s) in accordance with Art. 76 EPC:
**20724903.8 / 4 121 068**

(71) Applicant: **GammaDelta Therapeutics Limited**
**London W12 7FQ (GB)**

(72) Inventors:
- **SIMOES, Andre Goncalo do Espirito Santo**
  **London (GB)**
- **DI LORENZO, Biagio**
  **London (GB)**
- **KOSLOWSKI, Michael**
  **London (GB)**
- **SILVA-SANTOS, Bruno Miguel de Carvalho e**
  **London (GB)**
- **HUTTON, Andrew John**
  **London (GB)**

- **RECALDIN, Timothy Joel**
  **London (GB)**
- **FOWLER, Daniel**
  **London (GB)**
- **BROMLEY, Alice**
  **London (GB)**
- **NUSSBAUMER, Oliver**
  **London (GB)**

(74) Representative: **Sagittarius IP**
**Marlow International**
**Parkway**
**Marlow SL7 1YL (GB)**

Remarks:
•This application was filed on 19-11-2025 as a divisional application to the application mentioned under INID code 62.
•Claims filed after the date of filing of the application (Rule 68(4) EPC).

(54) **COMPOSITIONS FOR THE TREATMENT OF MYELOID MALIGNANCIES**

(57)    The invention relates to compositions comprising Vδ1+ T cells, for use in treating myeloid malignancies. The present invention also relates to methods of treatment using said compositions.

FIGURE 4

Processed by Luminess, 75001 PARIS (FR)

(52) Cooperative Patent Classification (CPC): (Cont.)

C-Sets
**A61K 31/675, A61K 2300/00;
A61K 31/7076, A61K 2300/00**

**Description**

**FIELD OF THE INVENTION**

The invention relates to compositions comprising Vδ1+ T cells, for use in treating myeloid malignancies. The present invention also relates to methods of treatment using said compositions.

**BACKGROUND OF THE INVENTION**

Acute myeloid leukaemia (AML) remains a clinical challenge due to frequent chemotherapy resistance and deadly relapses. AML has a poor (10%) survival rate among the elderly (age 65 or older), mostly due to resistance to standard treatment. Available treatment consists of a combination of cytarabine with an anthracyclin drug, which although effective at inducing complete remissions, ultimately selects for chemoresistant clones that drive refractory relapses. Promising alternatives to chemotherapy are targeted therapies and upcoming immunotherapies which have been successful against in B-cell malignancies.

Measurable residual disease (MRD) is an independent, postdiagnosis, prognostic indicator in AML and myelodysplastic syndrome (MDS) that is important for risk stratification and treatment planning, as patients who are MRD+ are more prone to relapse and have shorter survival rates even when morphological complete remission. Elimination of MRD in AML and MDS is an area of high unmet need but challenging due to lack of specific antigens expressed on leukemic blasts.

The presence of γδ T cells have been shown to have a positive correlation with prognosis in a number of solid and haematological cancers (Deniger et al. Clin. Cancer Res. (2014) 20(22): 5708-5719; Gentles et al. Nat. Med. (2015) 21(8): 938-945). While the use of Vδ2+ T cells in such treatments have been explored, the clinical manipulation of Vδ1+ T cells has been hindered by their relatively low abundance (<0.5%) among peripheral blood lymphocytes. However, methods such as those described in WO2016/198480, have recently provided improved yields of Vδ1+ T cells which may be suitable for clinical use to meet the need for treatment of myeloid malignancies for the first time as described herein.

**SUMMARY OF THE INVENTION**

According to a first aspect of the invention, there is provided an allogeneic composition comprising Vδ1+ T cells for use in the treatment of a patient with a myeloid malignancy.

According to a further aspect of the invention, there is provided a dose comprising the allogeneic composition for use as described herein.

According to a further aspect of the invention, there is provided a method of treating a myeloid malignancy comprising administering a therapeutically effective amount of an allogeneic composition comprising Vδ1+ T cells to a patient with said myeloid malignancy.

**BRIEF DESCRIPTION OF THE FIGURES**

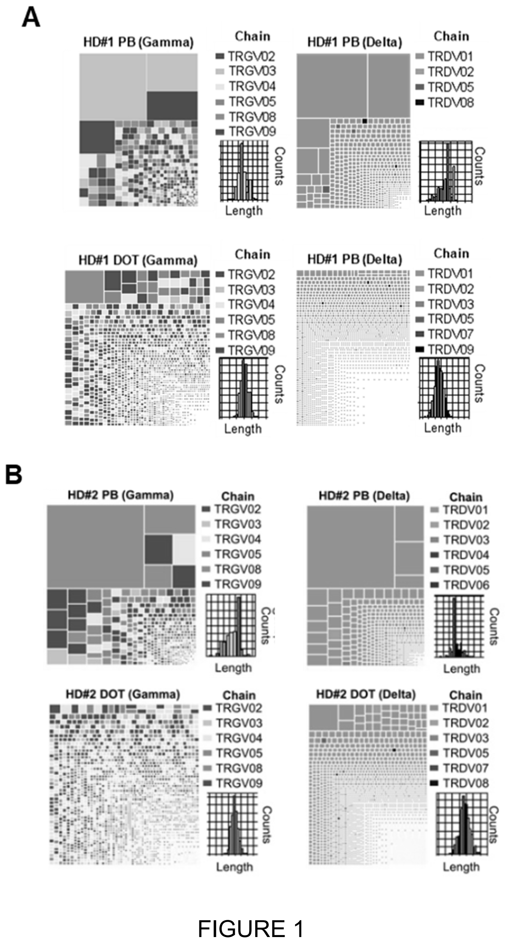

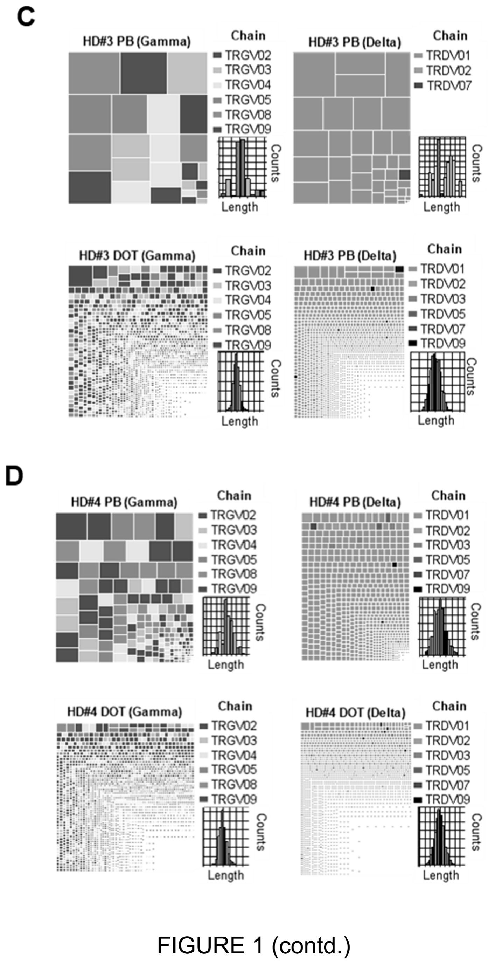

**Figure 1: γδ T cell composition displays higher clonal diversity than *ex vivo* Vδ1 T cells.** Graphical representation of TRGV and TRDV repertoires and CDR3 length (number of nucleotides) distribution of FACS-sorted Vδ1+ T cells from peripheral blood/PB or DOT-cell products derived from 4 independent healthy donors (HD# A-D). Each square represents a different clonotype (with distinct nucleotide sequence), its area is proportional to the relative abundance in the sample; and the colour groups the clonotypes by chains.

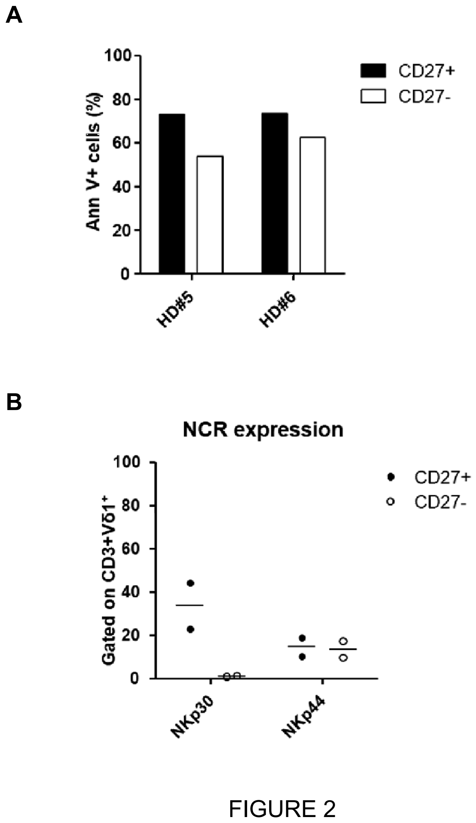

**Figure 2: Impact of CD27 expression phenotype on TCR repertoire diversity and AML reactivity of γδ T cell composition.** (A) *In vitro* killing of AML KG-1 cells by DOT-cells derived from pre-sorted CD27+ or CD27- Vδ1+ T cells (cultured for 21 days). Cells were co-incubated for 3 hours at 10:1 (E:T) ratio and then analysed by Annexin V staining (percentage of positive events among pre-labelled KG-1 cells). Data indicate the mean of two technical replicates for each donor. (B) NKp30 and NKp44 expression in CD27+ (black) and in CD27- (white) cells after DOT cells expansion. Indicated is mean of technical replicates.

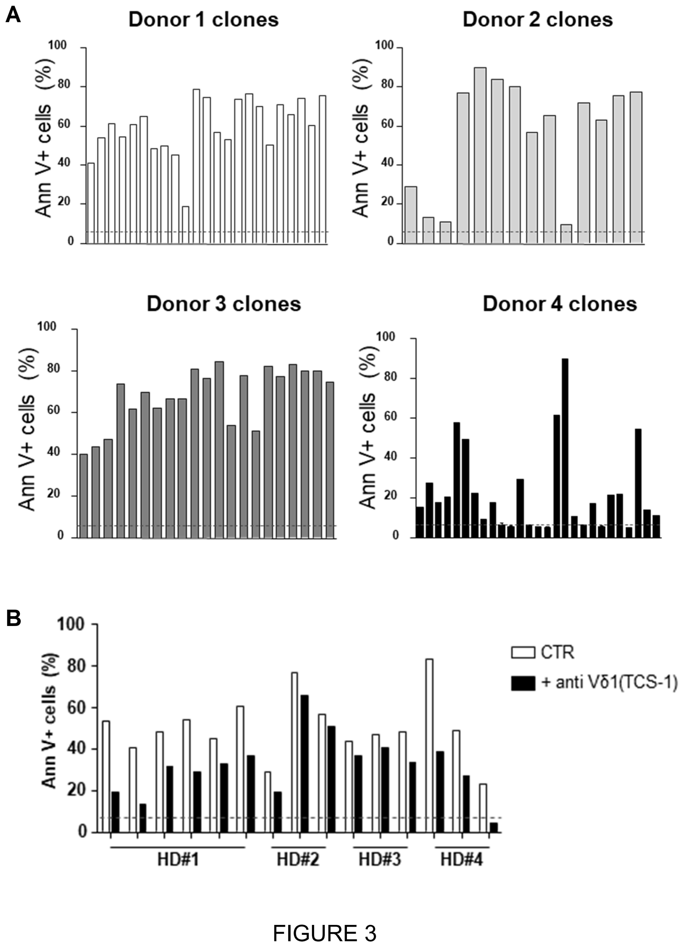

**Figure 3: Clonal γδ T cell composition reactivity against AML cells.** (A) and (B) show *in vitro* killing of AML KG-1 cells by DOT-cell clones generated from single Vδ1 T cells sorted from healthy donors. Cells were co-incubated for 3 hours at 10:1 (E:T) ratio and then analysed by Annexin V staining (percentage of positive events among pre-labelled KG-1 cells). Each bar represents killing of KG-1 cells upon coincubation with individual clones. Dashed horizontal line represents the mean basal tumour cell death (without DOT cells). In B, either anti-V$\delta$1 TCR-specific mAb or isotype control was added to the cultures. Shown are the clones where the blockade led to clearer reduction in KG-1 targeting. Data represent the average of two technical replicates and are derived from 4 independent healthy donors (HD).

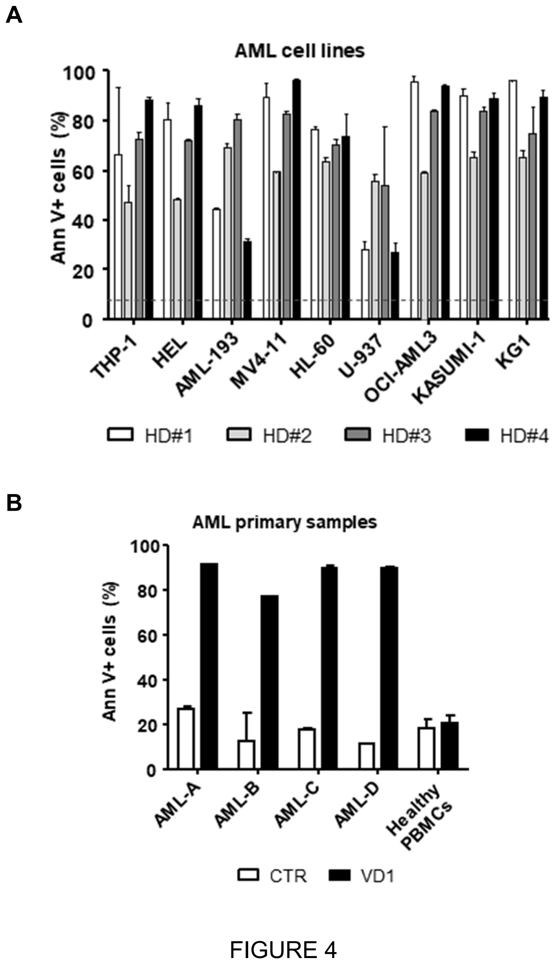

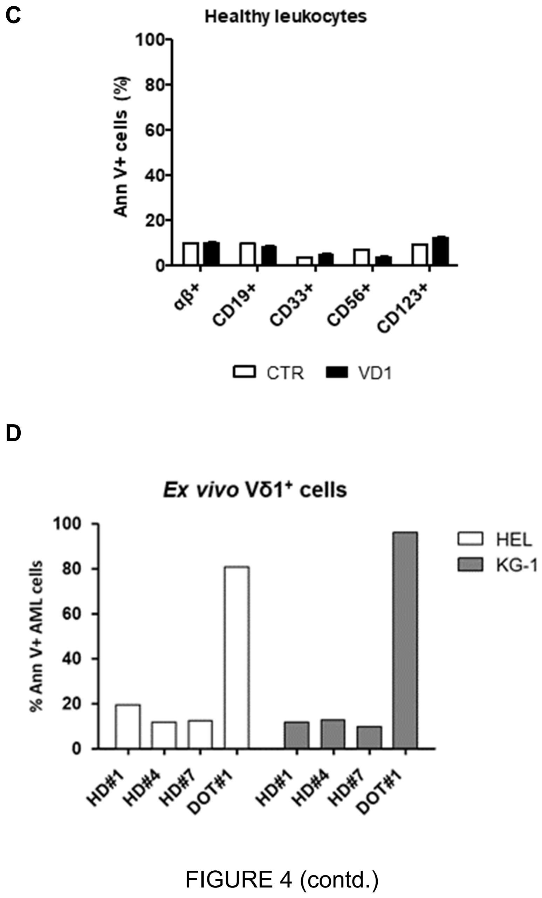

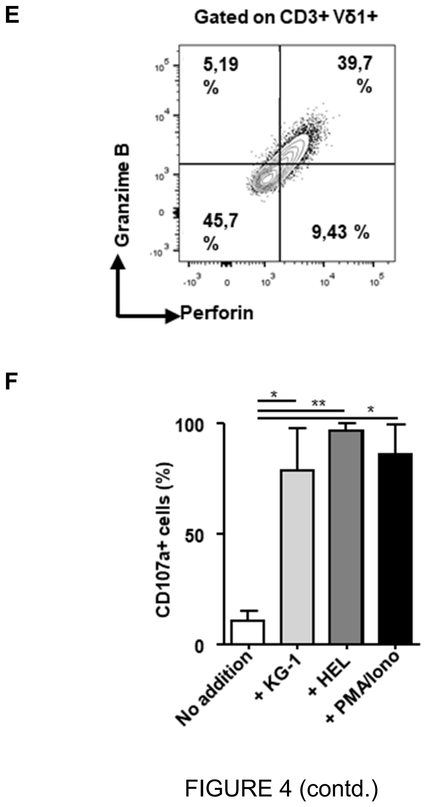

**Figure 4: $\gamma\delta$ T cell composition targets multiple AML cell types but not healthy leukocytes.** *In vitro* killing assays with DOT cells produced from 3-4 healthy donors, co-incubated for 3 hours at 10:1 (E:T) ratio with the indicated AML cell lines (A), primary AML samples (B), or normal leukocyte populations FACS-sorted from the peripheral blood (C). In A, the dashed horizontal line represents the mean basal tumour cell death; and in B, CTR refers also to tumour cells alone (without DOT cells). Experiments were performed with technical triplicates. (D) *In vitro* killing assays with unexpanded, fresh ("*ex vivo*") V$\delta$1+ T cells collected from 3 healthy donors, co-incubated for 3 hours at 10:1 (E:T) ratio with the indicated HEL or KG-1 cell lines. DOT-cells produced from HD#1 are shown as positive control. (E) DOT-cell expression of Granzyme B and Perforin as assessed by intracellular flow cytometry. (F) Percentage of CD107a+ DOT-cells after co-incubation with AML tumour targets; or upon PMA/ ionomycin stimulation (positive control); or no addition (negative control). Results are from two healthy donors, tested in duplicates.

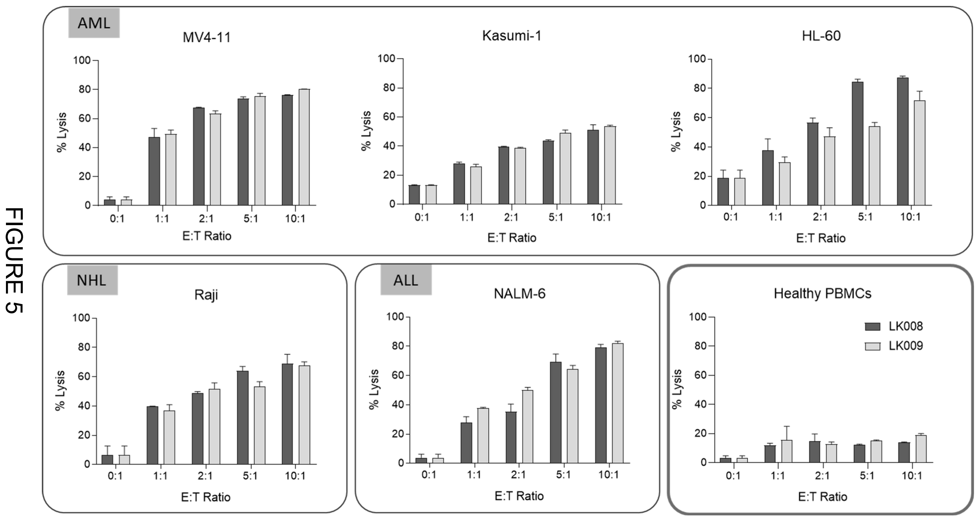

**Figure 5: V$\delta$1+ T cell cytotoxic activity against haematological tumour lines and sparing of healthy PBMCs.** 20 hour *in vitro* cytotoxicity assays of V$\delta$1+ T cells against a range of AML (MV4-11, Kasumi-1, HL-60), NHL (Raji) and ALL (NALM-6) tumour targets as well as healthy allogeneic PBMCs, across various effector : target ratios. Percentage target cell lysis is shown. N = 2.

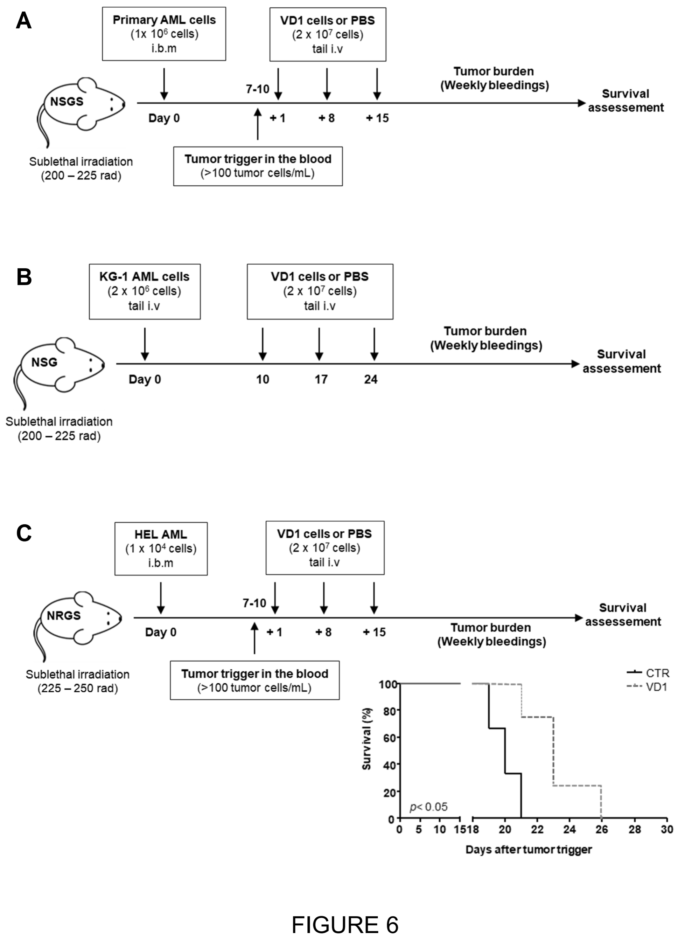

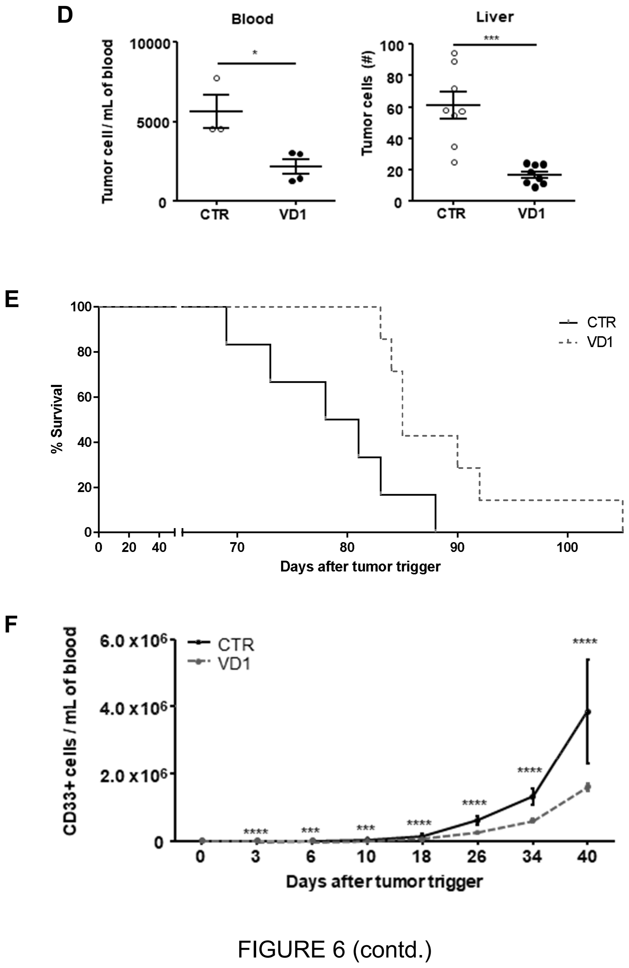

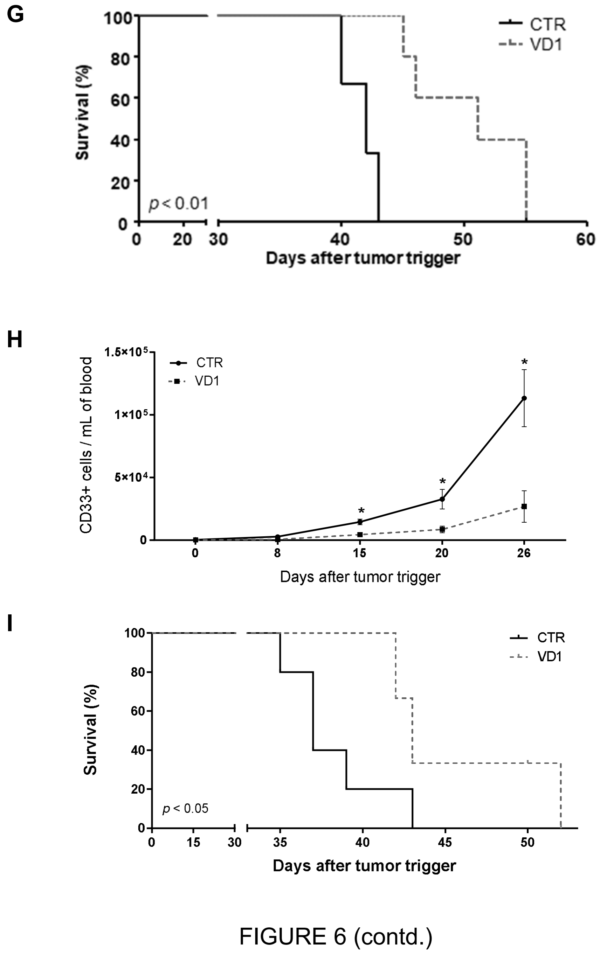

**Figure 6: *In vivo* AML targeting by $\gamma\delta$ T cell composition.** (A) Irradiated (200-225 rad) 8-12 week old NOD-SCID$\gamma$c$^{-/-}$-SGM3 (NSGS) mice were anaesthetized and subsequently transplanted in the right tibia (intra-bone marrow - i.b.m.) with 1 x 10$^6$ primary human AML cells. (B) Irradiated (200-225 rad) NSG 8-12 week old NOD-SCID$\gamma$c$^{-/-}$ (NSG) mice were injected intravenously (i.v.) with 2 x 10$^6$ human KG-1 cells. (C) Irradiated (225-250 rad) 8-12 weeks old NOD.Rag1-$\gamma$c$^{-/-}$-SGM3 (NRGS) mice were anaesthetized and subsequently transplanted in the right tibia (i.b.m.) with 1 x 10$^4$ human HEL cells. In (A) and (C), tumour engraftment was assessed through detection* of at least 100 tumour cells (tumour trigger) in the blood, 1-week after tumour cell injection. Treatments started as soon as 100 tumour cells were detected in the mouse blood (tumour trigger). In (B), treatments with either PBS or DOT cells started 10 days after intravenous injection of tumour cells. Animals were treated with three intravenous injections of PBS or 2 x 10$^7$ DOT cells, separated by 5 days. Survival curves for HEL-bearing NRGS hosts (n = 5 CTR, 4 DOT treated mice; p < 0.05). DOT cells (3 injections of 2 x 10$^7$ cells) were transferred to NSG mice (n = 6 CTR, 7 DOT-treated mice) preinjected with KG-1 AML cells (D-E); or NSGS mice (n = 5 CTR, 5 DOT-treated mice) bearing primary AML cells (F-G; patient-derived xenograft, PDX). Tumour burden was assessed in the blood and liver one week after the last DOT-cell transfer (D); or through weekly bleedings (F). Survival curves are presented in panels E (P < 0.05) and G (P < 0.01). (H-I) Second primary AML model was developed. (H) Tumour burden in the blood progression. (I) Survival curves for primary AML-bearing NSGS hosts (n = 5 CTR, n=5 DOT treated mice). Animals were sacrificed when advanced disease symptoms (such as back leg paralysis) were observed. Indicated are mean $\pm$ SEM; *, P < 0.05; ***, P < 0.001; ****, P < 0.0001.

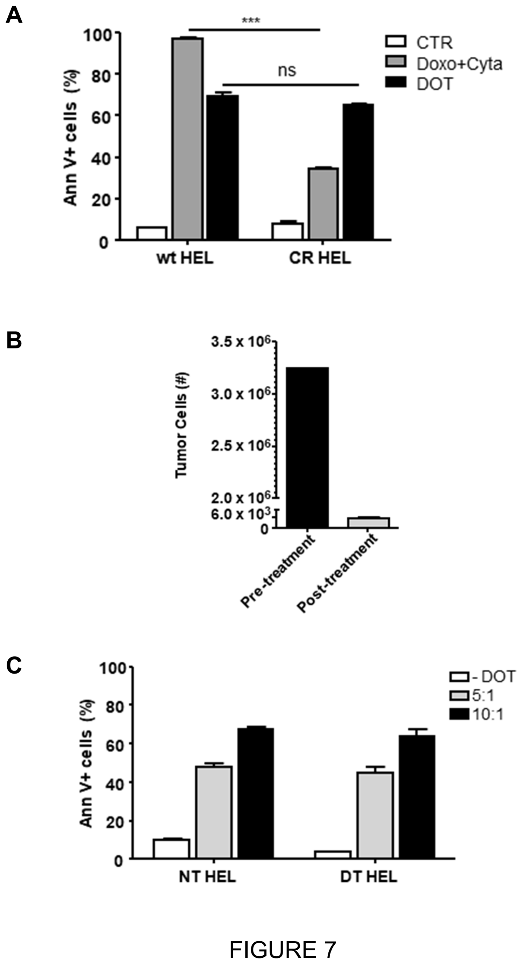

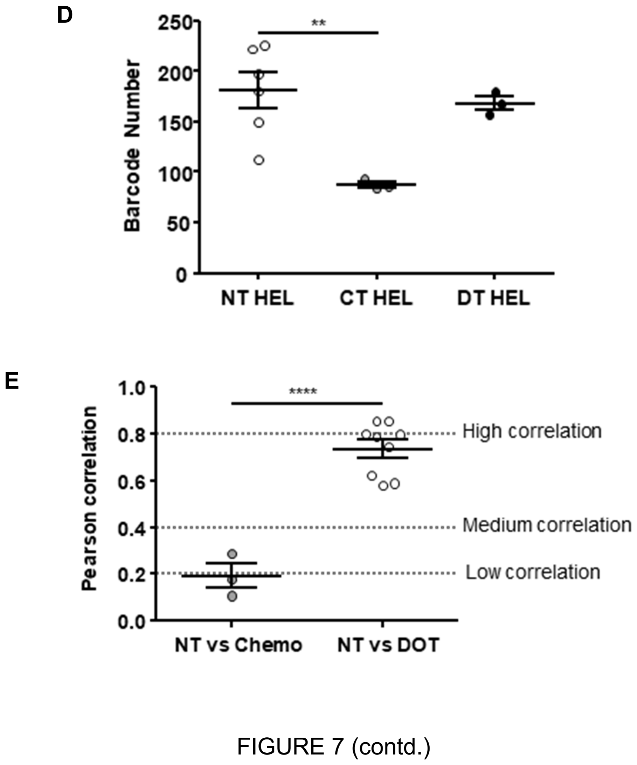

**Figure 7: $\gamma\delta$ T cell composition (re-)targets chemotherapy-resistant AML.** Comparison of the *in vitro* anti-AML activity of DOT cells and standard chemotherapy. (A) DOT cells and standard AML chemotherapy (doxorubicin plus cytarabine) protocols were tested against chemotherapy-naïve (wild type, wt) or chemo-relapsed (CR, regrown after >99% HEL cell elimination) AML cells. Shown are the percentages of Annexin V+ HEL cells after 3 hours of treatment. (B) Number of AML HEL cells before and after 72 hours of treatment with DOT cells (at 5:1 E:T ratio). Surviving cells (<1%) were resorted and allowed to regrow, thus generating the DOT-treated (DT) samples of (C-E). (C) DOT cells were co-incubated for 3 hours with nontreated (NT) or previously DOT-treated (DT) AML HEL cells at 5:1 or 10:1 (E:T) ratios. Shown are the percentages of Annexin V+ HEL cells. (D) Number of barcoded AML single-cell lineages in nontreated (NT), chemotherapy-treated (CT), or DOT-treated (DT) AML HEL cells. (E) Pearson correlation for distribution of barcoded AML single-cell lineages between different treatments. Dashed lines represent low (at 0.2), medium (at 0.4), and high (at 0.8) correlations, respectively. Indicated are mean $\pm$ SEM (**, P < 0.01; ***, P < 0.001; ****, P < 0.0001).

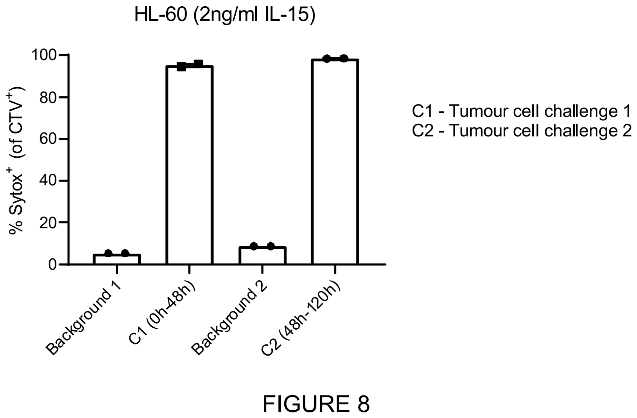

**Figure 8: Repeat cytotoxicity of expanded V$\delta$1 + T cell populations against haematological tumour lines.** The left side of the graph shows the percentage of CTV+ve events (HL-60 tumour targets) that were Sytox+ve, during challenge 1, while the right side of the graph shows the percentage of CTV+ve events that were Sytox+ve, during challenge 2. Mean with standard deviation of 2 donors.

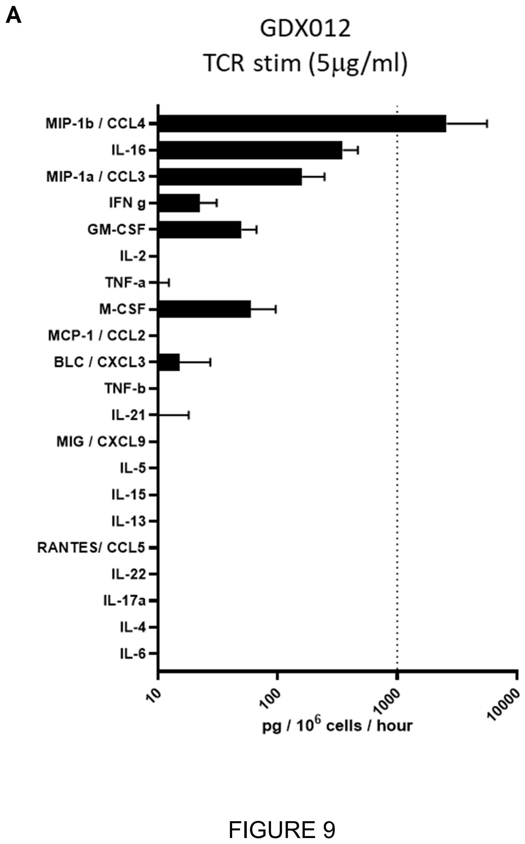

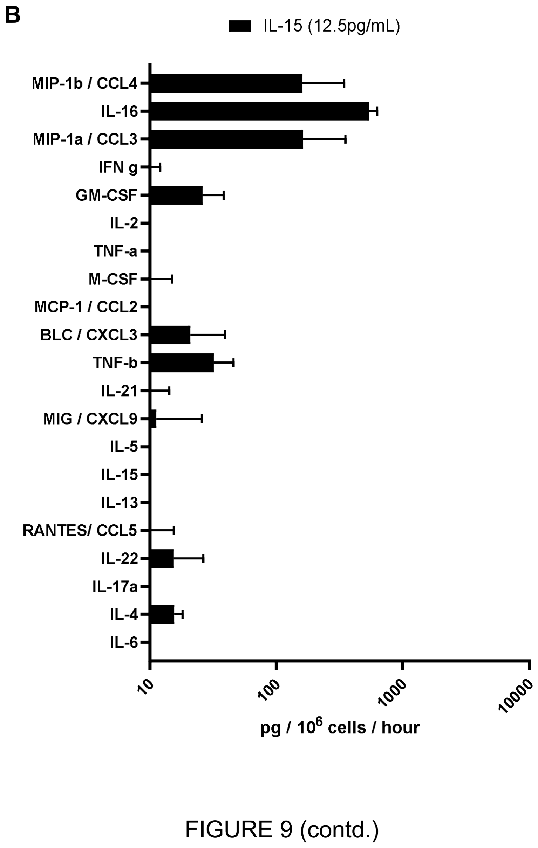

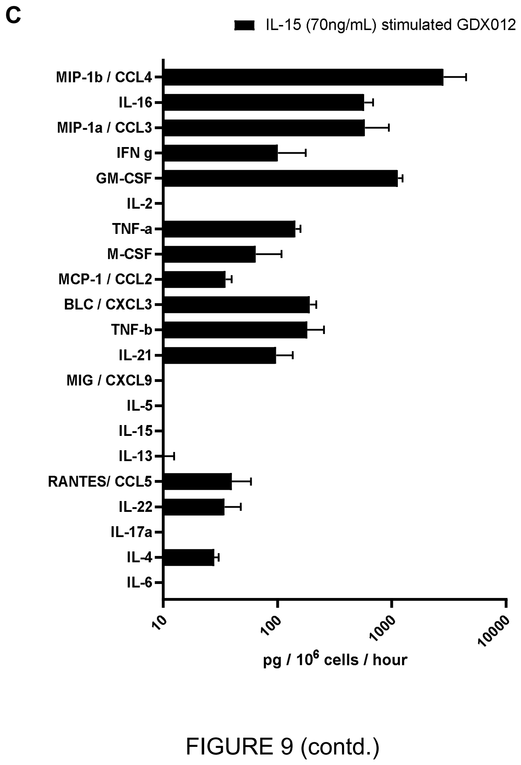

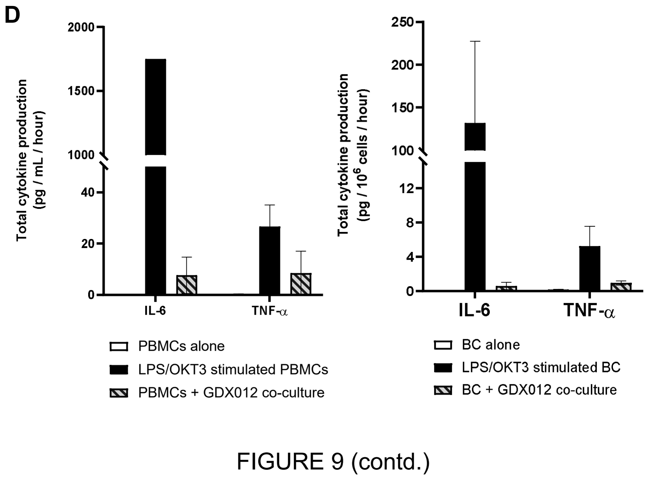

**Figure 9: Cytokine production by stimulated V$\delta$1-expanded cells.** Cytokine production (pg per million cells per hour) of Vδ1-expanded cells upon TCR stimulation (A). Pie chart representation of top cytokines produced by Vδ1-expanded cells stimulated by physiological levels of OKT3 and IL-15 (B) or super-physiological stimuli with IL-15 (C). IL-6 and TNFα production upon co-culture of blood samples (PBMCs or buffy coats) with Vδ1-expanded cells (D),

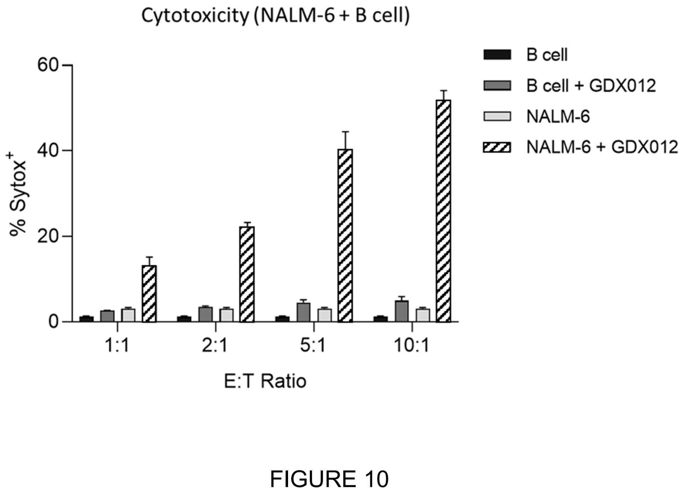

**Figure 10: Selective cytotoxic activity of expanded Vδ1+ T cell populations against NALM-6 cells and healthy B cells.** The graph shows both the percentage of CTV+ve events (healthy B cells) that were Sytox+ve, and the percentage CFSE+ve events (NALM-6 tumour cells) that were Sytox+ve, across the various E:T ratios. Mean and SD (technical duplicates). 1 experiment representative of 3 biological donors.

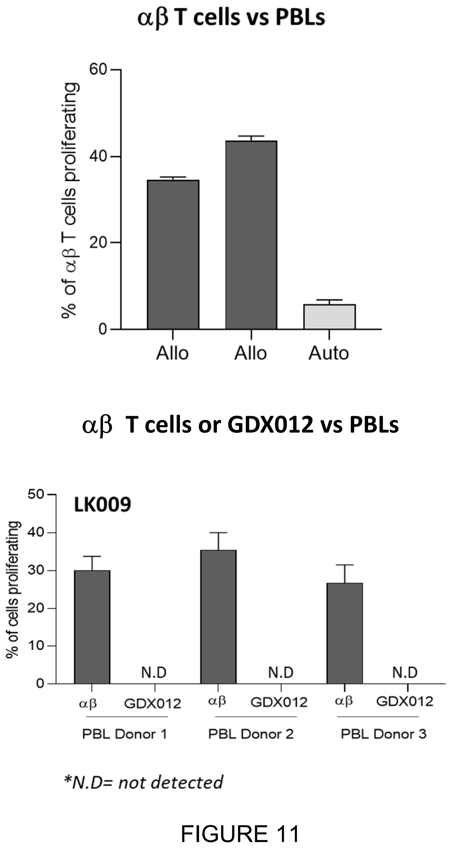

**Figure 11:** (A) PBLs isolated from buffy coat blood preparations and irradiated to arrest cell division potential were cocultured at a 1:1 ratio with CTV stained allogeneic or autologous blood T cell populations for 5 days without cytokine support. Cell division in response to co-culture with irradiated PBLs was then assessed via flow cytometric analysis of CTV dye dilution. Total % of αβ T cells divided is shown. N=3. (B) PBLs isolated from buffy coat blood preparations and irradiated to arrest cell division potential were cocultured at a 1:1 ratio with either CTV stained blood T cells or GDX012 cells prepared from two different donors (LK008, LK009). Blood T cells and GDX012 cells were derived from the same donor. Co-cultures were incubated for 5 days without cytokine support. Cell division in response to coculture with irradiated PBLs was then assessed via flow cytometric analysis of CTV dye dilution. Total % of αβ T cells (for blood T cells) or total % of live GDX023 cells divided is shown. Data shown in technical triplicates. N.D = not detected.

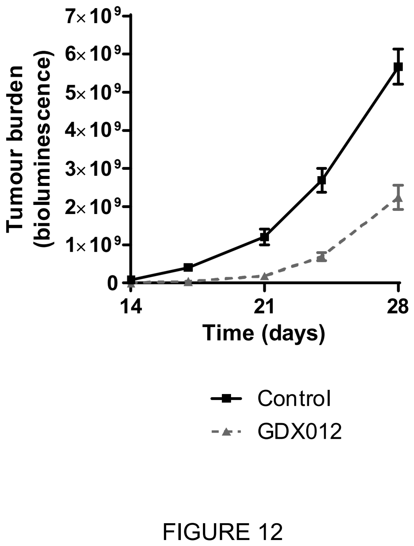

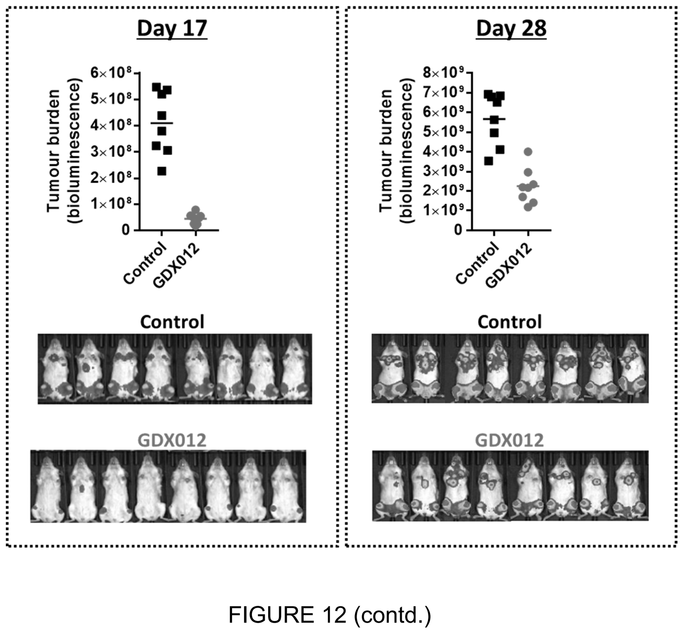

**Figure 12: Tumour Control in an *in vivo* model following a single intravenous administration of GDX012.** Tumour growth was tracked by whole body BLI in NSG mice challenged with an i.v. injection of $0.5\times10^6$ NALM-6-FLuc/GFP cells and then treated the next day with or without a single i.v. injection of $20\times10^6$ GDX012 cells. Control and treated mice all received i.p. injections of recombinant human IL-15 (1μg/mouse every 3 days for the duration of the study). Means $\pm$ SEMs (n=8) are shown between day 14 and 28 as well as individual data points and ventral whole body BLI images for the day 17 and day 28 time points.

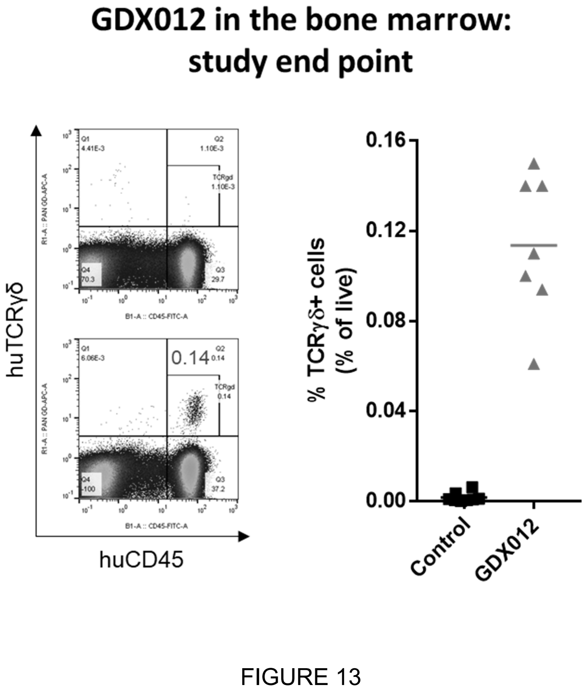

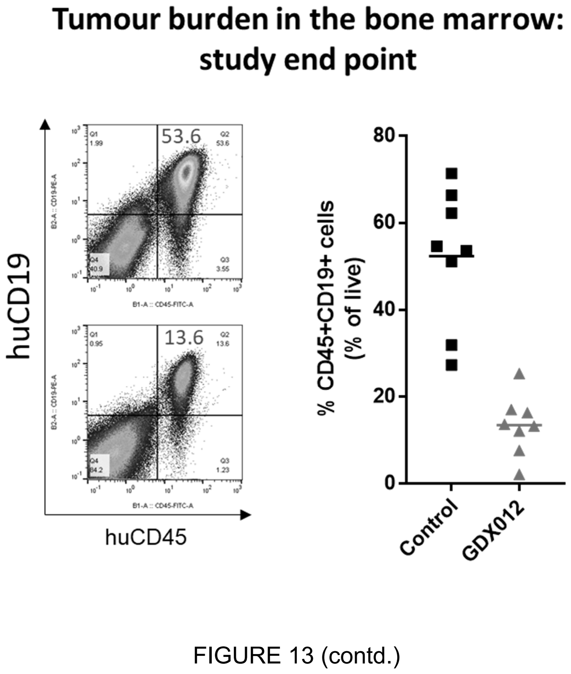

**Figure 13: Bone marrow homing in an *in vivo* model following a single intravenous administration of GDX012.** NSG mice challenged with an i.v. injection of $0.5\times10^6$ or $1\times10^6$ NALM-6-FLuc/GFP cells were treated with or without a single i.v. injection of $20\times10^6$ GDX012 cells either 24 hours or 6 days later. Control and treated mice all received i.p. injections of recombinant human IL-15 (1μg/mouse every 2-3 days for the duration of the study). The study was terminated after 4 weeks to assess GDX012 biodistribution and tumour burden in the bone marrow. Flow cytometry was performed on bone marrow from the hind limb long bones and the percentage of TCRγδ+ cells (GDX012) and CD19+ cells (NALM-6 cells) within live singlets was assessed. Representative flow cytometry plots and individual data points are shown.

## DETAILED DESCRIPTION OF THE INVENTION

According to a first aspect, there is provided an allogeneic composition comprising Vδ1+ T cells for use in the treatment of a patient with a myeloid malignancy. The data presented herein shows that Vδ1+ T cells expanded from allogeneic donors were highly polyclonal and devoid of dominant clones making them suitable as therapies for use in a wide range of donors. Further experiments have also shown that such compositions have limited potential for causing cytokine release syndrome and do not mediate mixed lymphocyte reactions which are important safety aspects when considering adoptive cell therapies. Additionally, the Vδ1+ T cells of the present invention are highly selective for and cytotoxic to myeloid cell lines and primary cells while sparing non-malignant 'healthy' cells of the same type.

### Myeloid malignancies

Myeloid malignancies are clonal diseases arising in hematopoietic stem or progenitor cells. They may be characterised by uncontrolled proliferation and/or blockage of differentiation of abnormal myeloid progenitor cells. Several mutations associated with these malignancies have been identified principally belonging to five classes: signalling pathways proteins (e.g. CBL, FLT3, JAK2, RAS), transcription factors (e.g. CEBPA, ETV6, RUNX1), epigenetic regulators (e.g. ASXL1, DNMT3A, EZH2, IDH1, IDH2, SUZ12, TET2, UTX), tumour suppressors (e.g. TP53), and components of the spliceosome (e.g. SF3B1, SRSF2) (Murati et al. (2012) BMC Cancer 12: 304).

The myeloid malignancy may comprise chronic (including myelodysplastic syndromes, myeloproliferative neoplasms and chronic myelomonocytic leukaemia) and acute (acute myeloid leukaemia) stages.

Based on the morphology, cytochemistry, immunophenotype, genetics, and clinical features of myeloid disorders, the World Health Organization (WHO) categorizes myeloid malignancies into five primary types: (1) acute myeloid leukaemia; (2) myelodysplastic syndromes; (3) myeloproliferative neoplasms; (4) myelodysplastic and myeloproliferative neoplasms; and (5) myeloid neoplasms associated with eosinophilia and abnormalities of growth factor receptors derived from platelets or fibroblasts. Classification is described further in Tefferi and Vardiman (2008) Leukemia 22:14-22.

Therefore, in one embodiment, the myeloid malignancy is selected from acute myeloid leukaemia (AML), myelodysplastic syndrome (MDS), myeloproliferative neoplasms (MPN), myelodysplastic and myeloproliferative (MDS/MPN) neoplasms and myeloid neoplasms associated with eosinophilia and abnormalities of growth factor receptors derived from platelets or fibroblasts. In a further embodiment, the myeloid malignancy is AML, MDS or MPN, in particular AML or MDS.

In one embodiment, the myeloid malignancy is AML. AML results from the clonal expansion of myeloid blasts in the peripheral blood, bone marrow or other tissue. It is caused when either the myeloid stem cells produce abnormal myeloblasts which do not become healthy white blood cells or too many myeloid stem cells become abnormal red blood cells or platelets. As a result, leukemic blasts, or immature cell forms, accumulate in the bone marrow, peripheral blood, and occasionally in other tissues, and the production of normal red blood cells, platelets, and mature granulocytes is reduced.

In an alternative embodiment, the myeloid malignancy is MDS. MDS and MPNs are often thought to be precursors to myeloid malignancies such as AML. Low blood cell counts, also referred to as "cytopenias", are a hallmark feature of MDS and are responsible for many of the symptoms associated with MDS, such as infection, anaemia, spontaneous bleeding, or easy bruising.

MDS types include refractory cytopenia with unilineage dysplasia (RCUD), refractory anaemia with ring sideroblasts (RARS) refractory cytopenia with multilineage dysplasia (RCMD), refractory anaemia with excess blasts (RAEB-1 and RAEB-2), myelodysplastic syndrome associated with isolated del (5q) and myelodysplastic syndrome unclassified (MDS-U). RCUD affects a single type of blood cell and can be divided into 3 subtypes: refractory anaemia (low numbers of red blood cells), refractory neutropenia (low numbers of white blood cells) and refractory thrombocytopenia (low numbers of platelets). RARS is similar to refractory anaemia, but there are a greater number of early red blood cells in the bone marrow that have a ring of iron in them (ring sideroblasts). RCMD affects more than one type of blood cell and is characterised by very few or no immature cells (blasts) in the blood and a small number of blasts in the bone marrow. For RAEB one or more blood cell levels are low, and many of these cells look abnormal in the bone marrow. In RAEB-2, there are more blast cells in the blood and bone marrow than in RAEB-1.

In one embodiment, the patient is positive for minimal residual disease (MRD+).

Minimal residual disease (MRD) refers to the presence of a small number of cancer cells in the body after cancer treatment. MRD is an independent, post-diagnosis, prognostic indicator in AML and MDS that is important for risk stratification and treatment planning.

Due to the low levels of cells, MRD requires testing using sensitive tests. The most widely used tests are flow cytometry, polymerase chain reaction (PCR) and next-generation sequencing (NGS) on samples of bone marrow cells and/or peripheral blood cells. Methods known in the art may be used to diagnose a patient with MRD. In one embodiment, the MRD+ patient is in complete remission, contains no detectable leukaemic blasts in the peripheral blood and/or contains less than 5% leukaemic blasts in the bone marrow.

The patient or subject to be treated is preferably a human cancer patient (e.g. a human cancer patient being treated for a blood cancer).

In one embodiment, the patient has previously been treated with chemotherapy. For example, the patient may have been treated with chemotherapy at least 3 days prior to administration of the allogeneic composition.

In one embodiment, the chemotherapy is selected from fludarabine and cyclophosphamide.

**Allogeneic composition**

In one embodiment, the allogeneic composition comprises at least about 90% CD45+ cells relative to total live cells. In a further embodiment, the allogeneic composition comprises at least about 91%, 92%, 93%, 94%, 95%, 96%, 97%, 98%, 99%, 99.5% CD45+ cells relative to total live cells.

In one embodiment, the allogeneic composition comprises at least about 60% $\gamma\delta$ T cells relative to total live cells. In a further embodiment, the allogeneic composition comprises at least about 70%, 75%, 80%, 85%, 90%, 95% $\gamma\delta$ T cells relative to total live cells.

In one embodiment, the allogeneic composition comprises an *ex vivo* expanded cell population enriched for V$\delta$1+ T cells relative to the starting unexpanded cell population. In one embodiment, the allogeneic composition comprises at least 10%, at least 20%, at least 30%, at least 40%, at least 50%, at least 60%, at least 70%, at least 80%, at least 90% of V$\delta$1+ T cells relative to total live cells. In a further embodiment, the allogeneic composition comprises greater than 30% V$\delta$1+ T cells relative to total live cells, for example at least 33%. In a further embodiment, V$\delta$1+ T cells comprise at least 30%, at least 40%, at least 50%, at least 60%, at least 70%, at least 80%, at least 90% or at least 95% of total $\gamma\delta$ T cells of the allogeneic composition. In further embodiment, V$\delta$1+ T cells comprise at least 40%, at least 50%, at least 60% of total $\gamma\delta$ T cells of the allogeneic composition.

In one embodiment, the allogeneic composition comprises less than 0.1% $\alpha\beta$ T cells relative to total live cells. Preferably the allogeneic composition comprises less than 0.09%, less than 0.08%, less than 0.07%, less than 0.06%, less than 0.05%, less than 0.04%, less than 0.03%, less than 0.02% or less than 0.01% $\alpha\beta$ T cells.

The allogeneic composition may comprise a dose that is suitable for administration to a patient. According to a further aspect, there is provided a dose of an allogeneic composition comprising V$\delta$1+ T cells for use in the treatment of a patient with a myeloid malignancy.

In one embodiment, a dose of the allogeneic composition comprises less than about $1 \times 10^{10}$ total live cells, such as less than about $9 \times 10^9$, $8 \times 10^9$, $7 \times 10^9$, $6 \times 10^9$, $5 \times 10^9$, $4 \times 10^9$, $3 \times 10^9$, $2 \times 10^9$, $1 \times 10^9$, $5 \times 10^8$, $3 \times 10^8$, $1 \times 10^8$, $5 \times 10^7$, $3 \times 10^7$, $1 \times 10^7$, $5 \times 10^6$, $3 \times 10^6$ or $1 \times 10^6$ total live cells. In one embodiment, a dose of the allogeneic composition comprises less than about $1 \times 10^8$ total live cells. In one embodiment, a dose of the allogeneic composition comprises more than about $1 \times 10^4$ total live cells, such as more than about $3 \times 10^4$, $5 \times 10^4$, $1 \times 10^5$, $3 \times 10^5$, $5 \times 10^5$, $1 \times 10^6$, $3 \times 10^6$, $5 \times 10^6$, $1 \times 10^7$, $3 \times 10^7$, or $5 \times 10^7$ total live cells. In one embodiment, a dose of the allogeneic composition comprises more than about $1 \times 10^6$ total live cells. In one embodiment, a dose of the allogeneic composition comprises between about $1 \times 10^4$ cells and about $1 \times 10^{10}$ total live cells, such as between about $1 \times 10^5$ total live cells and about $1 \times 10^9$ cells, in particular between about $1 \times 10^6$ cells and about $1 \times 10^8$ total live cells. In one embodiment, a dose of the allogeneic composition comprises between about $4 \times 10^7$, and $8 \times 10^9$, for example $4 \times 10^7$, $8 \times 10^7$, $4 \times 10^8$, $8 \times 10^8$, $1.2 \times 10^9$, $2.4 \times 10^9$, $4 \times 10^9$ or $8 \times 10^9$ total live cells.

The allogeneic composition may comprise a dose (such as a therapeutically effective dose) for administration a patient. In one embodiment, the patient is administered a dose of V$\delta$1+ T cells calculated per kg body weight of the patient. In some embodiments, a dose of V$\delta$1+ T cells as described herein comprises about $1 \times 10^5$, $5 \times 10^5$, $1 \times 10^6$, $1.5 \times 10^6$, $2 \times 10^6$, $3 \times 10^6$, $5 \times 10^6$, $1 \times 10^7$, $1.5 \times 10^7$, $2 \times 10^7$, $3 \times 10^7$, $5 \times 10^7$, $1 \times 10^8$, $2 \times 10^8$, or $5 \times 10^8$ cells/kg. In some embodiments, a dose of V$\delta$1+ T cells comprises at least about $1 \times 10^5$, $5 \times 10^5$, $1 \times 10^6$, $1.5 \times 10^6$, $2 \times 10^6$, $3 \times 10^6$, $5 \times 10^6$, $1 \times 10^7$, $1.5 \times 10^7$, $2 \times 10^7$, $3 \times 10^7$, $5 \times 10^7$, $1 \times 10^8$, $2 \times 10^8$, or $5 \times 10^8$ cells/kg. In some embodiments, a dose of V$\delta$1+ T cells comprises up to about $1 \times 10^6$, $1.5 \times 10^6$, $2 \times 10^6$, $3 \times 10^6$, $5 \times 10^6$, $1 \times 10^7$, $1.5 \times 10^7$, $2 \times 10^7$, $3 \times 10^7$, $5 \times 10^7$, $1 \times 10^8$, $2 \times 10^8$, or $5 \times 10^8$ cells/kg. In some embodiments, a dose of V$\delta$1+ T cells comprises about $1 \times 10^6$ - $1 \times 10^8$ cells/kg.

The dose of the allogeneic composition may comprise no more than $5 \times 10^4$ $\alpha\beta$ T cells/kg, such as no more than about $10^4$, $10^3$ or $10^2$ $\alpha\beta$ T cells/kg. Therefore, in one embodiment the dose comprises less than about $5 \times 10^4$ $\alpha\beta$ T cells/kg. In a further embodiment, the dose comprises less than about $1 \times 10^4$ $\alpha\beta$ T cells/kg.

In one embodiment, the allogeneic composition is frozen and then thawed before administration, In a further embodiment, the dose of the allogeneic composition is calculated prior to freezing. In another embodiment, the dose is calculated after thawing. In another embodiment, the allogeneic composition is not frozen.

As used herein, the term "about" when used herein includes up to and including 10% greater and up to and including 10% lower than the value specified, suitably up to and including 5% greater and up to and including 5% lower than the value specified, especially the value specified. The term "between", includes the values of the specified boundaries.

Pharmaceutical compositions may include expanded V$\delta$1+ T cell compositions as described herein in combination with one or more pharmaceutically or physiologically acceptable carrier, diluents, or excipients. Such compositions may include buffers such as neutral buffered saline, phosphate buffered saline and the like; carbohydrates such as glucose, mannose, sucrose or dextrans, mannitol; proteins; polypeptides or amino acids such as glycine; antioxidants; chelating agents such as EDTA or glutathione; adjuvants (e.g. aluminium hydroxide); and preservatives. Cryopreservation solutions which may be used in the pharmaceutical compositions of the invention include, for example, DMSO. Compositions can be formulated for any suitable administration, e.g. for intravenous administration.

In one embodiment, the pharmaceutical composition is substantially free of, e.g. there are no detectable levels of a contaminant, e.g. of endotoxin or mycoplasma.

**Gamma Delta T cells**

In one preferred embodiment, the $\gamma\delta$ T cells comprise a population of V$\delta$1+ T cells.

In some embodiments, the V$\delta$1+ T cells express CD27. For example, the V$\delta$1+ T cells may have a frequency of CD27+ cells of greater than 10%, greater than 20%, greater than 30%, greater than 40%, greater than 50%, greater than 60%, greater than 70%, greater than 80% or greater than 90%. Alternatively, the V$\delta$1+ T cells may have a frequency of CD27+ cells of about 10%, about 20%, about 30%, about 40%, about 50%, about 60%, about 70%, about 80% or about 90%. In certain embodiments, the V$\delta$1+ T cells have a frequency of CD27+ cells of greater than 10%. Thus, in one embodiment, the V$\delta$1+ T cells have a frequency of CD27+ cells of about 20%. In a further embodiment, the V$\delta$1+ T cells have a frequency of CD27+ cells greater than 20%. In one embodiment, the V$\delta$1+ T cells have a frequency of CD27+ cells of about 20%.

In some embodiments, the V$\delta$1+ T cells have a low proportion of cells expressing TIGIT. For example, the V$\delta$1+ T cells may have a frequency of TIGIT+ cells of less than 90%, less than 80%, less than 70%, less than 60%, less than 50%, less than 40%, less than 30%, less than 20% or less than 10%. Alternatively, the V$\delta$1+ T cells may have a frequency of TIGIT+ cells of about 90%, about 80%, about 70%, about 60%, about 50%, about 40%, about 30%, about 20% or about 10%. In certain embodiments, the Vδ1+ T cells have a frequency of TIGIT+ cells of less than 80%. Thus, in one embodiment, the Vδ1+ T cells have a frequency of TIGIT+ cells of about 70%. In a further embodiment, the Vδ1+ T cells have a frequency of TIGIT+ cells of less than 60%. In a yet further embodiment, the Vδ1+ T cells have a frequency of TIGIT+ cells of about 30%. Thus, in one embodiment the Vδ1+ T cells do not substantially express TIGIT.

In a further embodiment, the Vδ1+ T cells express CD27 and/or do not substantially express TIGIT.

**Methods of obtaining Vδ1+ T cell enriched compositions**

The Vδ1+ T cells may be obtained using methods known in the art. For example, the Vδ1+ T cells may be obtained using the methods described in WO2016/198480, WO2017/072367 or WO2018/202808, which are herein incorporated by reference. These methods may selectively expand Vδ1+ T cells (in particular, Vδ2- TCRγδ+ T cells) in culture. The methods are carried out on a sample, which may also referred to as a "starting sample". The methods can use either unfractionated samples or samples which have been enriched for TCRγδ+ T cells.

The data provided in the examples herein indicates that Vδ1+ T cell compositions expanded using exogenous growth factors have improved polyclonality compared to FACS-sorted, unexpanded Vδ1 T cells simply obtained from peripheral blood (i.e. *ex vivo* Vδ1 T cells), therefore in one embodiment, the allogeneic composition comprises Vδ1+ T cells obtained using an expansion method, in particular wherein said expansion method comprises culturing Vδ1+ T cells in the presence of exogenous growth factors.

The sample can be any sample that contains γδ T cells or precursors thereof including, but not limited to, blood, bone marrow, lymphoid tissue, epithelia, thymus, liver, spleen, cancerous tissues, lymph node tissue, infected tissue, fetal tissue and fractions or enriched portions thereof. The compositions and methods of the invention find particular use with Vδ1+ T cells obtained from haematological samples. Therefore, in one embodiment, the Vδ1+ T cells are obtained from a blood sample.

The sample is preferably blood including peripheral blood or umbilical cord blood or fractions thereof, including buffy coat cells, leukapheresis products, peripheral blood mononuclear cells (PBMCs) and low density mononuclear cells (LDMCs). In one embodiment, the blood sample is peripheral blood or a fraction thereof. In some embodiments the sample is human blood or a fraction thereof. The cells may be obtained from a sample of blood using techniques known in the art such as density gradient centrifugation. For example, whole blood may be layered onto an equal volume of FICOLL-HYPAQUE followed by centrifugation at 400xg for 15-30 minutes at room temperature. The interface material will contain low density mononuclear cells which can be collected and washed in culture medium and centrifuged at 200xg for 10 minutes at room temperature. The sample may be fresh or frozen.

In one embodiment, the Vδ1+ T cells are obtained from a human sample.

As described herein, the compositions and methods of the invention may be used with allogeneic derived Vδ1+ T cells, i.e. cells derived from a sample obtained from another donor. In one embodiment, the Vδ1+ T cells are obtained from a healthy donor.

Prior to culturing the sample or fraction thereof (such as PBMCs), the sample or fraction thereof may be enriched for certain cell types and/or depleted for other cell types. In one embodiment, the sample is enriched for T cells. The sample may be enriched for TCRγδ+ T cells. For example, the sample may be depleted of TCRαβ+ T cells, non-TCRγδ+ T cells and/or enriched for CD3+ cells. In one embodiment, the sample is first depleted of TCRαβ+ T cells, and then enriched for CD3+ cells.

The sample may be enriched or depleted of certain cell types using techniques known in the art. In one embodiment the cells of a particular phenotype may be depleted by culturing the sample or fraction thereof with an antibody cocktail containing antibodies that bind to specific molecules on the cells to be depleted. Preferably, the antibodies in the cocktail are coupled to magnetic microbeads that can be used to magnetically deplete or enrich target cells when these cells are forced to pass through a magnetic column. In one embodiment, the sample is depleted of αβ T cells.

Collection of the Vδ1+ T cells may include the physical collection of Vδ1+ T cells from the culture, isolation of the Vδ1+ T cells from other lymphocytes (e.g. αβ T cells, γδ T cells and/or NK cells) or isolation and/or separation of the Vδ1+ T cells from stromal cells (e.g. fibroblasts). In one embodiment, Vδ1+ T cells are collected by mechanical means (e.g. pipetting). In a further embodiment, Vδ1+ T cells are collected by means of magnetic separation and/or labelling. In a yet further embodiment, the Vδ1+ T cells are collected by flow cytometric techniques such as FACS. Thus, in certain embodiments, the Vδ1+ T cells are collected by means of specific labelling the Vδ1+ T cells. It will be appreciated that such collection of Vδ1+ T cells may include the physical removal from the culture, transfer to a separate culture vessel or to separate or different culture conditions.

Upon isolation from the sample, the Vδ1+ T cells will generally be part of a larger population of lymphocytes containing, for example, αβ T cells, B cells, and natural killer (NK) cells. In some embodiments, 0.1%-10% of the isolated population of lymphocytes are Vδ1+ T cells, e.g. 1-10% of the isolated population of lymphocytes are Vδ1+ T cells. In some embodiments, the percentage of Vδ1+ T cells is measured in proportion of CD45+ cells (leukocyte common antigen). In some embodiments, the isolated population is depleted of other cell types (e.g. depleted of αβ T cells). In some embodiments, the isolated population of CD45+ cells depleted of αβ T cells comprises at least 0.1% Vδ1+ T cells, such as at least 0.5% Vδ1+ T cells. In most cases, the γδ T cell population (e.g. blood-derived γδ T cell population) will include a large population of Vδ1 T cells. In some instances, less than 10% of the γδ T cells are Vδ2+ T cells (e.g. less than 10% of the γδ T cells are Vδ2+ T cells).

Once the cells in the sample have been fractionated and enriched, if desired, the cells may be cultured.

In certain embodiments, the invention features methods of expanding Vδ1+ T cells. These methods may be carried out *in vitro.* In some embodiments, the Vδ1+ T cells are expanded from a population of γδ T cells that has been isolated from a sample as described herein.

As used herein, references to "expanded" or "expanded population of Vδ1+ T cells" includes populations of cells which are larger or contain a larger number of cells than a non-expanded population. Such populations may be large in number, small in number or a mixed population with the expansion of a proportion or particular cell type within the population. It will be appreciated that the term "expansion step" refers to processes which result in expansion or an expanded population. Thus, expansion or an expanded population may be larger in number or contain a larger number of cells compared to a population which has not had an expansion step performed or prior to any expansion step. It will be further appreciated that any numbers indicated herein to indicate expansion (e.g. fold-increase or fold-expansion) are illustrative of an increase in the number or size of a population of cells or the number of cells and are indicative of the amount of expansion.

In one embodiment, the Vδ1+ T cells are obtained from a sample by a method comprising culturing the sample in a medium comprising a T cell mitogen and a growth factor having interleukin-4-like activity, in the absence of a growth factor having interleukin-15-like activity.

In one embodiment, the Vδ1+ T cells are obtained from a sample by a method comprising culturing the sample in a medium comprising a T cell mitogen and a growth factor having interleukin-15-like activity, in the absence of a growth factor having interleukin-4-like activity.

In one embodiment, the Vδ1+ T cells are obtained from a sample by a method comprising:

(1) culturing cells in the sample in a first culture medium comprising a T cell mitogen and a growth factor having interleukin-4-like activity; in the absence of a growth factor having interleukin-15-like activity; and
(2) culturing the cells obtained in step (1) in a second culture medium comprising a T cell mitogen and a growth factor having interleukin-15-like activity, in the absence of a growth factor having interleukin-4-like activity.

The terms "in the absence of interleukin-15, interleukin-2 and interleukin-7" and "in the absence of interleukin-4" refer not only to the complete absence of these cytokines in the culture medium, but also include the use of such cytokines at concentration levels so low that they cannot produce a measurable response or physiological effect in target cells and thus can be considered absent for practical purposes. Furthermore, "a measurable physiological effect in target cells" refers to any measurable change in the cells' physiological state according to standard definitions. For example, changes in the cell's physiological state can be detected by changes in their activation state (recognized by the up-regulation or downregulation of the expression levels of the early-activation cell marker CD69); or detected by changes in their differentiation state (recognized by the up-regulation or downregulation of NKG2D or NCRs), a few hours or a few days after contact with such cytokines. A measurable physiological effect may also be a change in the cell's proliferation rate, as measured by CFSE staining or by other techniques known in the art. It should be apparent for any one skilled in the art that cells cultured in the first culture medium must not receive a functionally relevant stimulus by IL-2, IL-7 and IL-15 or functionally similar growth factors. Additionally, cells in the second culture medium must not receive a functionally relevant stimulus by IL-4 or functionally similar growth factors. Preferably, these cytokines must not be present in the cell culture medium at a final concentration higher than 2 ng/ml; more preferably, not higher than 1 ng/ml, more preferably not higher than 0.1 ng/ml, more preferably, they should be absent.

The term "growth factor having interleukin-15-like activity " means any compound that has the same activity as IL-15 with respect to its ability to promote similar physiological effects on γδ T cells in culture and includes, but is not limited to, IL-15 and IL-15 mimetics, or any functional equivalent of IL-15, including IL-2 and IL-7. The physiological effects promoted by IL-15, IL-2 and IL-7 on cultured γδ T cells include the induction of cell differentiation towards a more cytotoxic phenotype, such as the upregulation of NKG2D and NCR (NKp30 and NKp44) expression levels, increased anti-tumour cytotoxic function and increased production of pro-inflammatory cytokines, such as IFN-y.

In one embodiment, the growth factor having interleukin-15-like activity is either interleukin-15 (IL-15), interleukin-2 (IL-2), or interleukin-7 (IL-7), preferably IL-15.

As used herein, "IL-15" refers to native or recombinant IL-15 or a variant thereof that acts as an agonist for one or more IL-15 receptor (IL-15R) subunits (e.g. mutants, muteins, analogues, subunits, receptor complexes, fragments, isoforms, and peptidomimetics thereof). IL-15, like IL-2, is a known T-cell growth factor that can support proliferation of an IL-2-dependent cell line, CTLL-2.

IL-15 can also refer to IL-15 derived from a variety of mammalian species, including, for example, human, simian, bovine, porcine, equine, and murine. An IL-15 "mutein" or "variant", as referred to herein, is a polypeptide substantially homologous to a sequence of a native mammalian IL-15 but that has an amino acid sequence different from a native mammalian IL-15 polypeptide because of an amino acid deletion, insertion or substitution. Variants may comprise conservatively substituted sequences, meaning that a given amino acid residue is replaced by a residue having similar physiochemical characteristics. Examples of conservative substitutions include substitution of one aliphatic residue for another, such as Ile, Val, Leu, or Ala for one another, or substitutions of one polar residue for another, such as between Lys and Arg; Glu and Asp; or Gln and Asn. Other such conservative substitutions, for example, substitutions of entire regions having similar hydrophobicity characteristics, are well known. Naturally occurring IL-15 variants are also encompassed by the invention. Examples of such variants are proteins that result from alternate mRNA splicing events or from proteolytic cleavage of the IL-15 protein, wherein the IL-15 binding property is retained. Alternate splicing of mRNA may yield a truncated but biologically active IL-15 protein. Variations attributable to proteolysis include, for example, differences in the N- or C-termini upon expression in different types of host cells, due to proteolytic removal of one or more terminal amino acids from the IL-15 protein (generally from 1-10 amino acids).

As used herein, "IL-2" refers to native or recombinant IL-2 or a variant thereof that acts as an agonist for one or more IL-2 receptor (IL-2R) subunits (e.g. mutants, muteins, analogues, subunits, receptor complexes, fragments, isoforms, and peptidomimetics thereof). Such agents can support proliferation of an IL-2-dependent cell line, CTLL-2 (33; American Type Culture Collection (ATCC®) **TIB** 214).

IL-2 can also refer to IL-2 derived from a variety of mammalian species, including, for example, human, simian, bovine, porcine, equine, and murine. Variants may comprise conservatively substituted sequences, meaning that a given amino acid residue is replaced by a residue having similar physiochemical characteristics. Examples of conservative substitutions include substitution of one aliphatic residue for another, such as Ile, Val, Leu, or Ala for one another, or substitutions of one polar residue for another, such as between Lys and Arg; Glu and Asp; or Gln and Asn. Other such conservative substitutions, for example, substitutions of entire regions having similar hydrophobicity characteristics, are well known. Naturally occurring IL-2 variants are also encompassed by the invention. Examples of such variants are proteins that result from alternate mRNA splicing events or from proteolytic cleavage of the IL-2 protein, wherein the IL-2 binding property is retained. Alternate splicing of mRNA may yield a truncated but biologically active IL-2 protein. Variations attributable to proteolysis include, for example, differences in the N- or C-termini upon expression in different types of host cells, due to proteolytic removal of one or more terminal amino acids from the IL-2 protein (generally from 1-10 amino acids).

As used herein, "IL-7" refers to native or recombinant IL-7 or a variant thereof that acts as an agonist for one or more IL-7 receptor (IL-7R) subunits (e.g. mutants, muteins, analogues, subunits, receptor complexes, fragments, isoforms, and peptidomimetics thereof). Mature human IL-7 occurs as a 152 amino acid sequence (less the signal peptide, consisting of an additional 25 N-terminal amino acids).

IL-7 can also refer to IL-7 derived from a variety of mammalian species, including, for example, human, simian, bovine, porcine, equine, and murine. Variants may comprise conservatively substituted sequences, meaning that a given amino acid residue is replaced by a residue having similar physiochemical characteristics. Examples of conservative substitutions include substitution of one aliphatic residue for another, such as Ile, Val, Leu, or Ala for one another, or substitutions of one polar residue for another, such as between Lys and Arg; Glu and Asp; or Gln and Asn. Other such conservative substitutions, for example, substitutions of entire regions having similar hydrophobicity characteristics, are well known. Naturally occurring IL-7 variants are also encompassed by the invention. Examples of such variants are proteins that result from alternate mRNA splicing events or from proteolytic cleavage of the IL-7 protein, wherein the IL-7 binding property is retained. Alternate splicing of mRNA may yield a truncated but biologically active IL-7 protein. Variations attributable to proteolysis include, for example, differences in the N- or C-termini upon expression in different types of host cells, due to proteolytic removal of one or more terminal amino acids from the IL-7 protein (generally from 1-10 amino acids).

The term "growth factor having interleukin-4-like activity" means any compound that has the same activity as IL-4 with respect to its ability to promote similar physiological effects on $\gamma\delta$ T cells in culture and includes, but is not limited to, IL-4 and IL-4 mimetics, or any functional equivalent of IL-4. The physiological effects promoted by IL-4 on $\gamma\delta$ T cells have been shown to include the decrease of NKG2D and NCR expression levels, the inhibition of cytotoxic function and improved selective survival. IL-4 has also been shown to significantly inhibit the secretion of pro-inflammatory cytokines, including IFN-y, TNF-$\alpha$, from activated TCR$\gamma\delta$+ T cells.

In one embodiment, the growth factor having interleukin-4-like activity is interleukin-4 (IL-4).

As used herein, "IL-4" refers to native or recombinant IL-4 or a variant thereof that acts as an agonist for one or more IL-4 receptor (IL-4R) subunits (e.g. mutants, muteins, analogues, subunits, receptor complexes, fragments, isoforms, and peptidomimetics thereof). Such agents can support differentiation of naïve helper T cells (Th0 cells) to Th2 cells. Mature human IL-4 occurs as a 129 amino acid sequence (less the signal peptide, consisting of an additional 24 N-terminal amino acids).

IL-4 can also refer to IL-4 derived from a variety of mammalian species, including, for example, human, simian, bovine, porcine, equine, and murine. Variants may comprise conservatively substituted sequences, meaning that a given amino acid residue is replaced by a residue having similar physiochemical characteristics. Examples of conservative substitutions include substitution of one aliphatic residue for another, such as Ile, Val, Leu, or Ala for one another, or substitutions of one polar residue for another, such as between Lys and Arg; Glu and Asp; or Gln and Asn. Other such conservative substitutions, for example, substitutions of entire regions having similar hydrophobicity characteristics, are well known. Naturally occurring IL-4 variants are also encompassed by the invention. Examples of such variants are proteins that result from alternate mRNA splicing events or from proteolytic cleavage of the IL-4 protein, wherein the IL-4 binding property is retained. Alternate splicing of mRNA may yield a truncated but biologically active IL-4 protein. Variations attributable to proteolysis include, for example, differences in the N- or C-termini upon expression in different types of host cells, due to proteolytic removal of one or more terminal amino acids from the IL-4 protein (generally from 1-10 amino acids).

In one embodiment, the V$\delta$1+ T cells are obtained from a sample by a method comprising:

(1) culturing cells in the sample in a first culture medium comprising a T cell mitogen and interleukin-4; in the absence of interleukin-15, interleukin-2 and interleukin-7; and
(2) culturing the cells obtained in step (1) in a second culture medium comprising a T cell mitogen and interleukin-15, in the absence of interleukin-4.

Methods of obtaining the V$\delta$1+ T cells from a sample may comprise additional growth factors. Therefore, in one embodiment, the first or second culture medium, or both culture media, further comprise one or more additional growth factors. Said additional growth factors may be selected from: interferon-$\gamma$ (IFN-y), interleukin-21 (IL-21), interleukin-1$\beta$ (IL-1$\beta$) and combinations thereof. Preferably, the additional growth factor is IFN-$\gamma$. These growth factors may be added to one or both culture media to further increase the expansion and purity levels of cultured V$\delta$1+ T cells. Additional growth factors may include IL-6, IL-7, IL-8, IL-9, IL-12, IL-18, IL-33, IGF-1, human platelet lysate (HPL), and stromal cell-derived factor-1 (SDF-1). In one embodiment, such factors are used in the expansion which selectively promote the expansion of V$\delta$1+ T cells.

The term "T cell mitogen" means any agent that can stimulate T cells through TCR signalling including, but not limited to, plant lectins such as phytohemagglutinin (PHA) and concanavalin A (ConA) and lectins of non-plant origin, antibodies that activate T cells, and other non-lectin/non-antibody mitogens. Preferred antibody clones include anti-CD3 antibodies such as OKT-3 and UCHT-1 clones, anti-$\gamma\delta$ antibodies such as B1 and IMMU510, or anti-V$\delta$1 antibodies. Within the context of the present invention, antibodies are understood to include monoclonal antibodies (mAbs), polyclonal antibodies, antibody fragments (e.g. Fab, and F(ab')2), single chain antibodies, single chain variable fragments (scFv) and recombinantly produced binding partners. In one embodiment, the antibody is an anti-CD3 monoclonal antibody (mAb). In another embodiment, the antibody is an anti-V$\delta$1 antibody. Other mitogens include phorbol 12-myristate-13-acetate (TPA) and its related compounds, such as mezerein, or bacterial compounds (e.g. Staphylococcal enterotoxin A (SEA) and Streptococcal protein A). The T cell mitogen may be soluble or immobilized and more than one T cell mitogen may be used in the method.

In one embodiment, the T cell mitogen is an antibody or a fragment thereof. The antibody or fragment thereof may be an anti-CD3 antibody, for example OKT-3. Alternatively, or additionally, the antibody or fragment thereof may be an anti-TCR$\gamma\delta$ antibody, such as a pan-$\gamma\delta$ TCR antibody or an anti-TCRV$\delta$1 antibody.

References herein to "culturing" include the addition of cells to a media comprising growth factors and/or essential nutrients required and/or preferred by the cells and/or non-haematopoietic tissue sample. Culturing may be by selective expansion, such as by choosing culturing conditions where V$\delta$1+ T cells are preferentially expanded over other cells types present in the sample. Alternatively, the expansion conditions are not selective and culturing may be followed by depletion of non-target cells (e.g. cells other than V$\delta$1+ T cells, such as $\alpha\beta$ T cells). Alternatively, the expansion conditions are not selective and depletion of non-target cells (e.g. cells other than V$\delta$1+ T cells, such as $\alpha\beta$ T cells) occurs prior to culturing.

In one embodiment, the culturing is performed in the absence of feeder cells.

In one embodiment, the culturing is performed in the absence of substantial stromal cell contact. In a further embodiment, the culturing is performed in the absence of substantial fibroblast cell contact.

In one embodiment, the V$\delta$1+ T cells are collected after at least 11 days of culturing, such as at least 14 days of culturing. In certain embodiments, the duration of culture according to the methods defined herein is at least 14 days. In certain embodiments, the duration of culture according to the methods defined herein is less than 45 days, such as less than 30 days, such as less than 25 days. In a further embodiment, the duration of culture according to the methods defined herein is between 14 days and 35 days, such as between 14 days and 21 days. In a yet further embodiment, the duration of culture according to the methods defined herein is about 21 days.

In further embodiments, the culturing is performed for a duration (e.g. at least 5 days, at least 6 days, at least 7 days, at least 8 days, at least 9 days, at least 10 days, at least 11 days, at least 12 days, at least 13 days, at least 14 days, at least 21 days, at least 28 days, or longer, e.g. from 5 days to 40 days, from 7 days to 35 days, from 14 days to 28 days, or about 21 days) in an amount effective to produce an expanded population of Vδ1+ T cells. In some embodiments, the culturing is for a period of several hours (e.g. about 2, 3, 4, 5, 6, 7, 8, 9, 10, 15, 18, or 21 hours) to about 35 days (e.g. 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, or 35 days). In one embodiment, the culturing is for a period of 14 to 21 days.

It will be understood that if two culture media are used the culturing in each media may occur for different lengths of time. For example, cells may be cultured in the first culture medium for a period of time ranging from about 2 days to about 21 days. More preferably, from about 3 days to about 14 days. More preferably, from about 4 days to 8 days. The cells may be cultured in the second culture medium for a period of time ranging from about 2 days to about 30 days. More preferably, from about 5 days to about 21 days. More preferably, from about 10 days to 15 days.

In one embodiment, the culturing is performed in a vessel comprising a gas permeable material. Such materials are permeable to gases such as oxygen, carbon dioxide and/or nitrogen to allow gaseous exchange between the contents of the vessel and the surrounding atmosphere. It will be appreciated that references herein to "vessel" include culture dishes, culture plates, single-well dishes, multi-well dishes, multi-well plates, flasks, multi-layer flasks, bottles (such as roller bottles), bioreactors, bags, tubes and the like. Such vessels are known in the art for use in methods involving expansion of non-adherent cells and other lymphocytes. Vessels comprising a gas permeable material have been found to increase the yield of isolated Vδ1+ T cells. Such vessels were also found to preferentially support Vδ1+ T cells and other lymphocytes over fibroblasts and other stromal cells (e.g. epithelial cells), including adherent cell-types. In a further embodiment, fibroblasts and/or other stromal cells (e.g. epithelial cells) are absent from cultures performed in vessels comprising a gas permeable material.

Such vessels comprising gas permeable materials may additionally comprise a gas permeable material that is non-porous. Thus, in one embodiment, the gas permeable material in non-porous. In some embodiments, the gas permeable material is a membrane film such as silicone, fluoroethylene polypropylene, polyolefin, or ethylene vinyl acetate copolymer. Furthermore, such vessels may comprise only a portion of gas permeable material, gas permeable membrane film or non-porous gas permeable material. Thus, according to a yet further embodiment, the vessel includes a top, a bottom and at least one sidewall, wherein at least part of the said vessel bottom comprises a gas permeable material that is in a substantially horizontal plane when said top is above said bottom. In one embodiment, the vessel includes a top, a bottom, and at least one sidewall, wherein at least a part of said bottom comprises the gas permeable material that is in a horizontal plane when said top is above said bottom. In a further embodiment, the vessel includes a top, a bottom and at least one sidewall, wherein the said at least one sidewall comprises a gas permeable material which may be in a vertical plane when said top is above said bottom, or may be a horizonal plane when said top is not above said bottom. It will be appreciated that in such embodiments, only a portion of said bottom or said side wall may comprise a gas permeable material. Alternatively, the entire of said bottom or entire of said sidewall may comprise a gas permeable material. In a yet further embodiment, said top of said vessel comprising a gas permeable material may be sealed, for example by utilisation of an O-ring. Such embodiments will be appreciated to prevent spillage or reduce evaporation of the vessel contents. Thus, in certain embodiments, the vessel comprises a liquid sealed container comprising a gas permeable material to allow gas exchange. In alternative embodiments, said top of said vessel comprising a gas permeable material is in the horizonal plane and above said bottom and is not sealed. Thus, in certain embodiments, said top is configured to allow gas exchange from the top of the vessel. In further embodiments, said bottom of the gas permeable container is configured to allow gas exchange from the bottom of the vessel. In a yet further embodiment, said vessel comprising a gas permeable material may be a liquid sealed container and further comprise inlet and outlet ports or tubes. Thus, in certain embodiments, the vessel comprising a gas permeable material includes a top, a bottom and optionally at least one sidewall, wherein at least a part of said top and said bottom comprise a gas permeable material and, if present, at least part of the at least one sidewall comprises a gas permeable material. Example vessels are described in WO2005/035728 and US9255243 which are herein incorporated by reference. These vessels are also commercially available, such as the G-REX® cell culture devices provided by Wilson Wolf Manufacturing, such as the G-REX6 well-plate, G-REX24 well-plate and the G-REX10 vessel.

In certain embodiments, the sample is cultured in media which is substantially free of serum (e.g. serum-free media or media containing a serum-replacement (SR)). Thus, in one embodiment, the sample is cultured in serum-free media. Such serum free medium may also include serum replacement medium, where the serum replacement is based on chemically defined components to avoid the use of human or animal derived serum. In an alternative embodiment, the sample is cultured in media which contains serum (e.g. human AB serum or fetal bovine serum (FBS)). In one embodiment, the sample is cultured in media which contains serum-replacement. In one embodiment, the sample is cultured in media which contains no animal-derived products.

It will be appreciated that embodiments wherein the sample is cultured in serum-free media have the advantage of avoiding issues with filtration, precipitation, contamination and supply of serum. Furthermore, animal derived products are not favoured for use in clinical grade manufacturing of human therapeutics.

Numerous basal culture media suitable for use in the proliferation of γδ T cells are available, in particular medium, such as AIM-V, Iscoves medium and RPMI-1640 (Life Technologies). The medium may be supplemented with other media factors as defined herein, such as serum, serum proteins and selective agents, such as antibiotics. For example, in some embodiments, RPMI-1640 medium containing 2 mM glutamine, 10% FBS, 10 mM HEPES, pH 7.2, 1% penicillin-streptomycin, sodium pyruvate (1 mM; Life Technologies), non-essential amino acids (e.g. 100 $\mu$M Gly, Ala, Asn, Asp, Glu, Pro and Ser; 1X MEM non-essential amino acids (Life Technologies)), and 10 $\mu$l/L $\beta$-mercaptoethanol. In an alternative embodiment, AIM-V medium may be supplemented with CTS Immune serum replacement and amphotericin B. Conveniently, cells are cultured at 37°C in a humidified atmosphere containing 5% $CO_2$ in a suitable culture medium during isolation and/or expansion.

Examples of other ingredients that may be added to the culture media, include, but are not limited to, plasma or serum, purified proteins such as albumin, a lipid source such as low density lipoprotein (LDL), vitamins, amino acids, steroids and any other supplements supporting or promoting cell growth and/or survival.

The V$\delta$1+ T cells obtained according to the described methods can be separated from other cells that may be present in the final culture using techniques known in the art including fluorescence activated cell sorting, immunomagnetic separation, affinity column chromatography, density gradient centrifugation and cellular panning.

The obtained V$\delta$1+ T cells may be immediately used in the therapeutic, experimental or commercial applications described herein or the cells may be cryopreserved for use at a later date.

## Methods of Treatment

According to a further aspect of the invention, there is provided a method of treating a myeloid malignancy comprising administering a therapeutically effective amount of an allogeneic composition comprising V$\delta$1+ T cells to a patient with said myeloid malignancy.

The term "therapeutically effective amount" as used herein means an amount effective, at dosages and for periods of time necessary to achieve the desired results.

As described hereinbefore, the myeloid malignancy may be selected from acute myeloid leukaemia (AML) and myelodysplastic syndrome (MDS). The invention finds particular use in patients who are positive for minimal residual disease (MRD+).

In one embodiment, the method additionally comprises administration of chemotherapy.

In one embodiment, the patient is treated with chemotherapy at least 3 days prior to administration of the allogeneic composition. The chemotherapy may be selected, for example, from fludarabine and cyclophosphamide.

In one embodiment, the patient is administered a dose of V$\delta$1+ T cells calculated per kg body weight of the patient. In some embodiments, the therapeutically effective amount comprises about $1 \times 10^5$, $5 \times 10^5$, $1 \times 10^6$, $1.5 \times 10^6$, $2 \times 10^6$, $3 \times 10^6$, $5 \times 10^6$, $1 \times 10^7$, $1.5 \times 10^7$, $2 \times 10^7$, $3 \times 10^7$, $5 \times 10^7$, $1 \times 10^8$, $2 \times 10^8$, or $5 \times 10^8$ cells/kg. In some embodiments, the therapeutically effective amount comprises about $1 \times 10^6$, $1.5 \times 10^6$, $2 \times 10^6$, $3 \times 10^6$, $5 \times 10^6$, $1 \times 10^7$, $1.5 \times 10^7$, $2 \times 10^7$, $3 \times 10^7$, $5 \times 10^7$, $1 \times 10^8$, $2 \times 10^8$, or $5 \times 10^8$ cells/kg. In some embodiments, the therapeutically effective amount comprises up to about $1 \times 10^6$, $1.5 \times 10^6$, $2 \times 10^6$, $3 \times 10^6$, $5 \times 10^6$, $1 \times 10^7$, $1.5 \times 10^7$, $2 \times 10^7$, $3 \times 10^7$, $5 \times 10^7$, $1 \times 10^8$, $2 \times 10^8$, or $5 \times 10^8$ cells/kg. In some embodiments, the therapeutically effective amount comprises about $1 \times 10^6$-$1 \times 10^8$ cells/kg. In one embodiment, the therapeutically effective amount comprises less than about $1 \times 10^8$ cells/kg.

In some embodiments, the therapeutically effective amount comprises less than about $1 \times 10^{10}$ total live cells, such as less than about $1 \times 10^9$ total live cells or less than about $1 \times 10^8$ total live cells. In some embodiments, the therapeutically effective amount comprises about $8 \times 10^9$, $4 \times 10^9$, $2.4 \times 10^1$, $1.2 \times 10^9$, $8 \times 10^8$, $4 \times 10^8$, $8 \times 10^7$ or $4 \times 10^7$ total live cells.

In some embodiments, the therapeutically effective amount comprises less than about $5 \times 10^4$ $\alpha\beta$ T cells/kg. In a further embodiment, the therapeutically effective amount comprises less than about $1 \times 10^4$ $\alpha\beta$ T cells/kg.

In one embodiment, the subject receives an initial administration of V$\delta$1+ T cells (e.g. an initial administration of $10^6$ to $10^8$ V$\delta$1+ T cells per kg body weight of the subject, e.g. $10^6$ to $10^7$ V$\delta$1+ T cells per kg body weight of the subject), and one or more (e.g. 2, 3, 4, or 5) subsequent administrations of V$\delta$1+ T cells. In one embodiment, the one or more subsequent administrations are administered less than 15 days, e.g. 14, 13, 12, 11, 10, 9, 8, 7, 6, 5, 4, 3, or 2 days after the previous administration, e.g. less than 4, 3, or 2 days after the previous administration.

In some embodiments, one or more additional therapeutic agents can be administered to the subject. The additional therapeutic agent may be selected from the group consisting of an immunotherapeutic agent, a cytotoxic agent, a growth inhibitory agent, a radiation therapy agent, an anti-angiogenic agent, or a combination of two or more agents thereof. The additional therapeutic agent may be administered concurrently with, prior to, or after administration of the expanded V$\delta$1+ T cells. The additional therapeutic agent may be an immunotherapeutic agent, which may act on a target within the subject's body (e.g. the subject's own immune system) and/or on the transferred V$\delta$1+ T cells.

The administration of the compositions may be carried out in any convenient manner. The compositions described herein may be administered to a patient transarterially, subcutaneously, intradermally, intratumorally, intranodally, intramedullary, intramuscularly, by intravenous injection, or intraperitoneally, e.g. by intradermal or subcutaneous injection. In particular, the compositions are administered as an intravenous infusion.

It will be understood that all embodiments described herein may be applied to all aspects of the invention.

Certain aspects and embodiments of the invention will now be illustrated by way of example and with reference to the figures described above.

**EXAMPLES**

**Materials and Methods**

Ethics statement

Primary Acute Myeloid Leukaemia (AML) cells were obtained from the peripheral blood of patients at first presentation, after informed consent and institutional review board approval. The study was conducted in accordance with the Declaration of Helsinki.

Mice

NOD SCID $\gamma_c^{-/-}$ (NSG), NOD SCID $\gamma_c^{-/-}$ SGM3 (NSGS), and NOD Rag1$^{-/-}$ $\gamma_c^{-/-}$ SGM3 (NRGS) mice were obtained from the Jackson Laboratories. Age and sex-matched mice were randomly distributed among the different groups. Disease development was followed through weekly bleedings (in intrabone marrow models) and disease end-point is achieved upon first indication of back leg decreased mobility. All animal procedures were performed in accordance to national guidelines from the Direção Geral de Veterinária and approved by the Animal Ethics Committee of Instituto de Medicina Molecular João Lobo Antunes (Lisboa, Portugal).

γδ T cell composition and TCR repertoire analysis

The γδ T cell composition was produced using methods described in WO2016/198480. In particular, the "Delta One T" (DOT) cell protocol refers to the research-scale version of the expansion protocol as described in Almeida et al. (2016) Clin. Cancer Res. 22: 5795-804, while "GDX012" refers to the larger scale version of the expansion protocol using larger vessels, e.g. G-Rex vessels. In brief, MACS-sorted γδ T cells were resuspended in serum-free culture medium (OpTmizer-CTS) supplemented with 5% autologous plasma and 2 mmol/L L-glutamine (Thermo Fisher Scientific). Animal-free human cytokines recombinant IL-4 [rIL4] (100 ng/mL), recombinant interferon-γ [rIFNy] (70 ng/mL), recombinant IL-21 [rIL21] (7 ng/mL), and recombinant IL-1β [rIL1β] (15 ng/mL), and a soluble mAb to CD3 (clone OKT-3, 70 ng/mL), were added to the medium. Cells were incubated at 37°C and 5% $CO_2$, fed at regular intervals with fresh medium including recombinant IL-15 [rIL15] (70 ng/mL), IFNy (30 ng/mL), and anti-CD3 (1 mg/mL). The cells are optionally frozen after expansion and thawed before use.

For TRGV and TRDV repertoire analysis, Vδ1+ T cells were FACS-sorted either from the initial blood sample (*ex vivo*); or from the final DOT-cell product generated as described above. Next-generation sequencing was performed as described previously (Verstichel et al. (2017) Sci. Immunol. 2: eaah4232; Ravens et al. (2017) Nat. Immunol.18: 393-401; Di Lorenzo et al. (2019) Sci Data 6: 115). For DOT-cell clone generation, CD3+ TCRVδ1+ TCRVδ2- single cells were FACS-sorted into 96-wells/plates; and cultured for 21 days using the DOT-cell protocol as described above in the presence of (weekly renewed) $10^4$ irradiated autologous peripheral blood mononuclear cells (feeders).

AML cell targeting *in vitro* and *in vivo*

AML cell lines (THP-1, HEL, AML-193, MV4-11, HL-60, U-937, OCI-AML3, Kasumi-1, and KG-1) were obtained from and authenticated by the German Resource Center for Biologic Material (DSMZ); and used at passages p3-p8. Lentiviral barcoding of AML cells was performed and analysed as detailed previously (Naik et al. (2013) Nature 496: 229-232). For *in vitro* targeting, AML cell lines or primary samples were co-incubated with DOT cells for 3 hours; and stained with Annexin V, as detailed previously (Nobrega-Pereira et al. (2018) Cancer Res.78: 731-741). For *in vivo* targeting, three xenograft hAML models were established as represented in **Figures 6A-C.** The patient-derived xenograft (intratibial injection) was described previously (12). Tumour burden was assessed by staining with antihuman CD45 (HI30) and CD33 (P67.6). Flow cytometry acquisition was performed on an LSR Fortessa (BD Biosciences) and data was analysed with FlowJo X software (Tree Star).

Statistical analysis

Performed using GraphPad Prism software. All data expressed as mean ± SEM. Comparisons of two groups by Student t test; and more than two groups by ANOVA test with Dunnet post test. Animal survival comparisons performed using log-rank (Mantel-Cox) test.

**EXAMPLE 1: γδ T cell composition displays higher clonal diversity than *ex vivo* Vδ1+ T cells.**

The γδ T cell product was initially characterised upon expansion of αβ-depleted peripheral blood mononuclear cells with the DOT-cell protocol described in the Materials and Methods section. Because reports described the clonal expansion and focusing of the adult peripheral blood Vδ1+ T-cell repertoire, likely driven by common pathogens such as cytomegalovirus (CMV), the effect of the expansion on the TCR repertoire was analysed. Next-generation sequencing was performed of the CDR3 regions in TRGV and TRDV genes, before and after the cells were 3 weeks in culture. Expanded Vδ1+ cells were found to be highly polyclonal and devoid of dominant clones, in contrast to fresh unexpanded *ex vivo* Vδ1 T cells from all donors analysed (**Figures 1A-D**).

This was illustrated by the contribution of the top 20 expanded clones to the overall Vδ1 TCR repertoire. Although these 20 clones represented >60% in the peripheral blood, they accounted for less than 10% in the Vδ1+ T-cell products. Moreover, few clonotypes (especially for TRDV) were shared between those identified unexpanded ex vivo cells and in expanded Vδ1+ cells (**Table 1**).

Table 1: Clonotype counts before *(ex vivo)* or after expansion and number of shared clonotypes.

|  | γ chain | | | δ chain | | |
|---|---|---|---|---|---|---|
|  | *Ex vivo* | **Expanded** | **Shared** | *Ex vivo* | **Expanded** | **Shared** |
| **HD#1** | 488 | 2706 | 57 | 775 | 7376 | 11 |
| **HD#2** | 561 | 1734 | 63 | 602 | 4791 | 20 |
| **HD#3** | 29 | 3844 | 4 | 50 | 4591 | 0 |
| **HD#4** | 222 | 6868 | 28 | 807 | 9592 | 7 |

The basis for the diversification of the expanded Vδ1+ T cell repertoire was investigated. Given the previous association of CD27 downregulation with pre-expanded/differentiated Vδ1+ T cells, the TCR clonality of expanded cells produced from pre-sorted CD27- versus CD27+ subsets was compared and shown to display distinct proliferation capacities under the Vδ1+ T-cell expansion protocol. It was found that the generation of diverse Vδ1+ T cells after expansion was restricted to CD27+ precursors. Moreover, the Vδ1+ T cell population (generated from bulk Vδ1 T cells) was shown to be largely composed of CD27+ cells. Vδ1+ T-cell products originating from pre-sorted CD27+ cells expressed NKp30 and were highly cytotoxic against KG-1 AML cells (**Figures 2A-B**).

**EXAMPLE 2: γδ T cell composition reactivity against AML cells**

To assess the functional relevance of Vδ1+ T cell polyclonality, clones were generated from single-cell sorted Vδ1+ T cells, expanded/differentiated using an adapted DOT-cell expansion protocol including the addition of feeder cells. Cytotoxicity of these cells was tested against the AML cell line KG-1 (**Figure 3A**). Most clones (from different donors) were found to be efficient at inducing apoptosis of KG-1 cells upon short (3-hour) coincubation in vitro (**Figure 3A**). These results show that the expanded Vδ1+ T cell population is composed of multiple clones with intrinsic capacity to target AML cells. To functionally test whether the TCR is involved in this reactivity, the killing assay was performed in the presence of a Vδ1 TCR-specific blocking mAb (or isotype control), and only a mild reduction in KG-1 cell targeting across a number of clones from different donors was observed (**Figure 3B**).

To further evaluate the anti-AML activity, bulk DOT-cell products from multiple donors were tested against various other AML cell lines as well as primary samples obtained from patients at diagnosis. In all cases, the expanded Vδ1+ T cell population readily (within 3 hours) killed AML cells in vitro (**Figures 4A-B**), in similar fashion to what was reported for CAR-T cells (Mardiros et al. (2013) Blood 122: 3138-3148; Gill et al. (2014) Blood 123: 2343-2354; Petrov et al. (2018) Leukemia 32: 1317-1326), and unlike unexpanded fresh *ex vivo* Vδ1 T cells (**Figure 4D**). Cytotoxicity was associated with increased degranulation and expression of perforin and granzyme B upon tumour cell recognition (**Figures 4E-F**). The expanded Vδ1+ T cells did not target any normal leukocyte population (myeloid or lymphoid) from the peripheral blood of healthy volunteers **(Figure 4C),** including CD33+ and CD123+ myeloid progenitor cells, whose on-target depletion by the respective CAR-T cells is known to be responsible for the unwanted myeloablation.

Allogeneic γδ T cell compositions were also tested against other haematological tumour cell lines.

PBMC Generation from Buffy Coats

Buffy coats were diluted 1 part blood with 3 parts PBS, and layered onto Leucosep tubes (20ml buffy coat-PBS mix per tube). Leucosep tubes were spun down for 20 mins at RT, 2000rpm (approx. 800g) with the centrifuge brake set to 1. The interface was collected and combined into one tube, washed one further time in PBS and then used in downstream assays.

GDX012 cells ("effectors") derived from 2 donors were run in flow cytometry cytotoxicity assays against the following tumour and healthy lines ("targets"):

- NALM-6
- Raji
- MV4-11
- Kasumi
- HL-60
- Healthy allogeneic peripheral blood mononuclear cells

Target cells were washed with PBS and stained with CellTrace Violet (CTV) for 20 minutes at room temperature. After 20 minutes, cells were washed with medium containing at least 10% serum and resuspended in target cell medium (RPMI) without cytokines. Subsequently, target cells were co-cultured with effector cells at 10:1, 5:1, 2:1 and 1:1 (effector : target) ratios in duplicate or triplicate for 20 hours at 37°C. The assay was run in the absence of cytokines.

After 20 hours, dead cells were stained by the addition of SytoxAADvanced to the culture medium for 10 minutes at room temperature and immediately analysed on the MACSQuant10. Percentage lysis was calculated using the formula:

$$\% \ lysis = 100 - \frac{CTV^+ Sytox^- \ (in \ test \ condition)}{total \ CTV^+ cells \ (in \ the \ targets \ alone \ condition)} * 100$$

Overall, all tumour lines were sensitive V$\delta$1+ T cell-mediated targeting. Higher E:T ratios led to greater levels of cytotoxicity. Conversely, the healthy PBMCs were completely spared, regardless of the E:T ratio. Thus, expanded V$\delta$1+ T cells are capable of targeting a broad range of haematological tumour targets while sparing healthy allogeneic cells. Results are shown in **Figure 5.**

### EXAMPLE 3: Xenograft models for *in vivo* AML targeting by $\gamma\delta$ T cell composition

To test DOT expanded V$\delta$1+ T cells against AML *in vivo,* various independent xenograft models of AML were established (**Figures 6A-C**). Both in AML cell line models (**Figures 6C-E**) and in two patient-derived xenografts (**Figures 6F-I**), administration of DOT-expanded V$\delta$1+ T cells reduced tumour burden and increased host survival, without noticeable toxicity. Although CAR-T cells have been reported to produce bigger survival benefits in AML xenografts (Mardiros *et al.* (2013); Gill *et al.* (2014); Petrov *et al.* (2018)), these models were biased to AML cell lines uniformly expressing the target antigens. On the other hand, the toxicity of a strategy predicted to induce myeloablation in patients cannot be evaluated with the use of xenografts. This data supports the combined safety and efficacy profiles of the VD1+ enriched $\gamma\delta$ T cell composition makes it a candidate for adoptive cell therapy of AML.

### EXAMPLE 4: $\gamma\delta$ T cell composition targets chemotherapy-resistant AML

Chemoresistance drives deadly relapses in the context of AML therapies, therefore DOT expanded V$\delta$1+ T cells were evaluated for targeting chemoresistant AML cells. For that purpose, AML cells were treated with cytarabine plus doxorubicin for 72 hours, which led to >99% tumour cell elimination, before allowing surviving cells to regrow, and then treating the culture with chemotherapy or V$\delta$1+ T cells. Although the cytotoxic efficacy of chemotherapy was reduced, the targeting efficacy of V$\delta$1+ T cells was unaffected (**Figure 7A**), demonstrating the superior capacity of V$\delta$1+ T cells to target chemoresistant AML cells.

In light of this, and taking into account the polyclonal and multi-reactive V$\delta$1+ T-cell repertoire (shown in **Figure 1**), the inventors questioned the ability of V$\delta$1+ T cells to retarget AML cells following a first V$\delta$1+ T cell treatment that eliminated >99% tumour cells in 72 hours (**Figure 7B**). The remaining, approximately 0.1% of AML cells present at 72 hours were FACS-sorted and allowed to regrow before retreatment with V$\delta$1+ T cells. V$\delta$1+ T cells killed pre-treated AML cells as efficiently as nontreated controls (**Figure 7C**), suggesting that V$\delta$1+ T -cell treatment did not select for a specific subset of V$\delta$1+ T resistant AML cells. To track the AML clonal dynamics upon therapeutic (V$\delta$1+ T cells or chemotherapy) pressure, single AML cells were tagged with cellular barcodes (non-coding DNA sequences that can be tracked by NGS). Although chemotherapy selectively targeted approximately half of all the barcoded AML single-cell lineages, V$\delta$1+ T cells preserved the clonal architecture of the AML population (**Figures 7D-E**).

These data collectively suggest that the breadth of AML targeting by expanded V$\delta$1+ T cells avoids the selection of resistant lineages and allows efficient retreatment. Emergence of refractory relapses after chemotherapy needs to be prevented. This work thus provides evidence for clinical application of the $\gamma\delta$ T cell composition in AML treatment.

**EXAMPLE 5: Repeat cytotoxicity of V$\delta$1+ T cell populations**

Repeated Challenge Cytotoxicity Assay

To determine the capacity of V$\delta$1+ T cells for repeat cytotoxicity against suitable tumour cell lines, GDX012-expanded V$\delta$1+ T cells derived from 2 donors were run in flow cytometry cytotoxicity assays against AML HL-60 target cells. Briefly, HL-60 Target cells were washed with PBS and stained with CellTrace Violet (CTV) for 20 minutes at room temperature. After 20 minutes, cells were washed with medium containing at least 10% serum and resuspended in target cell medium (RPMI) without cytokines. Subsequently, target cells were co-cultured with effector cells at a 10:1 (effector : target) ratio in duplicate or triplicate for 48 hours at 37°C. The assay was run in the presence of 2ng/ml IL-15. After 48 hours, dead cells were stained by the addition of SytoxAADvanced to the culture medium for 10 minutes at room temperature and immediately analysed on the MACSQuant10. Percentage Sytox+ve was calculated by quantifying the percentage of CTV+ve cells that were positive for the SytoxAADvanced dye.

For the second, or repeat, killing assay, unused wells from the first killing assay were harvested via vigorous pipetting, spun down (300g, 5 minutes), supernatant removed, and resuspended in fresh Target Cell Culture Media. Cells were counted and added to new wells. Fresh HL-60 cells, stained with CTV as before, were resuspended and added to the newly plated effector cells, again at a 10:1 effector : target ratio. Wells were re-supplemented with fresh IL-15 at 2ng/ml and left for a further 72 hours. Killing of target cells was quantified using SytoxAADvanced as described above. Results are shown in **Figure 8.**

Overall, the HL-60 tumour line was sensitive to two rounds of Vd1 cell-mediated targeting, across 5 days, in the context of IL-15 cytokine supplementation, and thus, the expanded Vd1 cells are capable of providing prolonged tumour cell targeting capacity.

**EXAMPLE 6: $\gamma\delta$ T cell composition has limited potential for cytokine release syndrome**

Cytokine release syndrome (CRS) is a key safety issue with other immunotherapies, such as $\alpha\beta$ T cell therapies. To assess the potential risk for cytokine burst of our product, cytokine levels were measured in the supernatants of cryopreserved GDX012 cells thawed and cultured for 21 hours in several distinct conditions. In fact, physiological stimulation of GDX012 cells either through the TCR (**Figure 9A**) or with IL-15, known to induce potent responses by V$\delta$1+ T cells, (**Figure 9B**) induces the release of mainly Th1-related cytokines and barely detectable levels of key cytokines responsible for CRS. Even under super-physiological stimuli with IL-15 (**Figure 9C**), the levels of IL-6 are undetectable during the course of our assay whereas some levels of TNF$\alpha$ start to be seen. This behaviour indicates that there is an advantageous safety profile for the claimed composition. In addition, further studies demonstrated the very limited risk for cytokine release burst upon co-culture of GDX012 cells with allogeneic blood derived samples, PBMCs and buffy coats, (**Figure 9D**), with practically undetectable levels of IL-6 and TNF$\alpha$ after 21 hours of co-culture.

**EXAMPLE 7: $\gamma\delta$ T cell composition spares allogeneic B cells**

To determine the selectivity of the cells, GDX012 expanded V$\delta$1+ T cells derived from 3 donors were run in flow cytometry cytotoxicity assays against a mixture of CFSE-labelled NALM-6 cells (tumourigenic B cells) and CTV-labelled B cells (non-tumourigenic primary B cells).

Isolation of primary B cells

100E6 PBMCs were taken from a freshly received buffy coat and centrifuged at 300g for 7 minutes. Supernatant was removed and cells were resuspended in 40 $\mu$l MACS Buffer / 10 $\mu$l Pan B Cell Biotin-Antibody Cocktail per $10^7$ cells. The cell suspension was left in the fridge for 5 minutes. 30 $\mu$l MACS Buffer / 20 $\mu$l anti-biotin microbeads per $10^7$ cells was added to the cell suspension and left in the fridge for 10 minutes. Meanwhile, an LS column, inserted into the quadroMACS on a magnetic stand, was equilibrated by passing 3 ml of MACS buffer through the column. The cell suspension was applied to the column and the effluent collected. A wash of 3 ml of MACS buffer was applied to the column and collected. This represented the negatively enriched B cell fraction.

Cytotoxicity Assay

B cells were washed with PBS and stained with CellTrace Violet (CTV) for 20 minutes at room temperature. NALM-6 cells were washed with PBS and stained with CFSE for 20 minutes at room temperature. After 20 minutes, cells were washed with medium containing at least 10% serum and resuspended in target cell medium (RPMI) without cytokines. Subsequently, target cells were co-cultured with effector cells at 10:1:1, 5:1:1, 2:1:1 and 1:1:1 (effector : NALM-6 : B cell) ratios in duplicate or triplicate for 20 hours at 37°C. The assay was run in the absence of cytokines. After 20 hours, dead cells were stained by the addition of SytoxAADvanced to the culture medium for 10 minutes at room temperature and immediately analysed on the MACSQuant10. Percentage Sytox+ve was calculated by quantifying the percentage of CTV+ve or CFSE+ve cells that were positive for the SytoxAADvanced dye. Results are shown in **Figure 10.**

Overall, the NALM-6 cells were appreciably targeted whilst the healthy B cells were completely spared. Targeting of the NALM-6 cells was dependent on the E:T ratio. Higher E:T ratios led to greater levels of cytotoxicity. Conversely, the healthy B cells were completely spared, regardless of the E:T ratio. Thus, expanded Vd1+ T cells specifically target B cell tumours without causing any collateral damage to healthy B cells cultured in the same plate.

**EXAMPLE 8: $\gamma\delta$ T cell composition does not mediate a mixed lymphocyte reaction (MLR)**

In order to demonstrate a culture system suitable for the detection of allogeneic responses, donor blood T cells were isolated, CTV stained and cultured with irradiated peripheral blood lymphocytes (PBLs) from either autologous or allogenic sources. Cultures were run for 5 days after which $\alpha\beta$ T cells division was assessed via flow cytometric analysis of CTV dye-dilution. Methods are provided herein.

Results from this experiment clearly show in **Figure 11A** that irradiated PBLs can elicit a robust allogeneic response from blood T cells in a mixed lymphocyte response culture system, while autologous-matched cultures showed a greatly reduced level of T cell proliferation. This indicates the suitability of this culture system in addressing alloreactive potential of a given T cell population

To address the alloreactive potential of expanded V$\delta$1+ T cells, GDX012 cells were cultured with irradiated PBLs from allogeneic donors. As a control, matched blood T cells from the same individual the GDX012 product was derived from were CTV stained and cultured against irradiated PBLs from the same allogeneic donors. Cultures were run for 5 days after which cell division was assessed via flow cytometry.

Results shown in **Figure 11B** indicated that blood $\alpha\beta$ T cells clearly divided in the presence of allogeneic PBLs, while GDX012 cells (expanded V$\delta$1+ T cells) failed to persist in culture in any significant numbers. The elicited $\alpha\beta$ response to the allogeneic PBLs demonstrate T cell proliferation typical of a mixed lymphocyte reaction. Despite this mismatch, GDX012 cell did not proliferate in the presence of the same irradiated PBLs, indicating that GDX012 cells are unable to mount allogeneic responses in the same manner as $\alpha\beta$ T cells. Taken together, these results indicate that GDX012 is unable to mediate GvHD in the same manner as $\alpha\beta$ T cells.

Preparation of buffy coats, depletion of CD14 events and irradiation of resultant peripheral blood lymphocytes

Buffy coat blood underwent density gradient separation to isolate the PBMC fraction. A small portion of these resultant PBMCs were frozen in 10%Cryostor10 cryopreservation medium and frozen at -80°C. The remaining PBMCs were washed, labelled for human CD14 and CD14 depletion carried out using Miltenyi MACS LS columns. The resultant PBL fraction was then cultured in complete RPMI media (RPMI media containing 10% fetal calf serum, 1% Penicillin/-streptomycin, 1% HEPES, 1% non-essential amino acids and 1% sodium pyruvate) overnight at 37°C, 5%$CO_2$.

The next day, PLBs were harvested via pipetting and exposed to 40Gy x-ray irradiation to arrest cell proliferation potential. These cells represent the "stimulator" cell fraction in the MLR assays.

Isolation of blood T cells and setup of MLR plates

PBMCs from either buffy coat sources or from refrozen leukopak material were taken from frozen storage and thawed. PBMCs were then washed, labelled with pan T cell isolation beads and blood T cells isolated via MACS LS columns. Resultant blood T cell fractions were then washed and stained with Cell Tracker Violet (CTV). CTV+ blood T cells were then co-cultured with irradiated stimulator PBLs. In parallel, frozen vials of GDX12 were defrosted, washed and immediately stained with CTV. CTV+ GDX012 cells were then cocultured with irradiated stimulator PBLs. In all cases, a ratio of 1:1 effector-stimulator cells were setup per well. Co-cultures were setup in complete RPMI media. Cultures were then incubated at 37°C, 5% $CO_2$ for 5 days. Cultures were not fed with extra media after this initial setup.

**EXAMPLE 8: Expanded V$\delta$1+ T cell composition prevent tumour growth *in vivo***

A cell line derived xenograft model was used to assess the biodistribution and efficacy of GDX012 *in vivo.* Immunodeficient NOD SCID gamma (NSG) mice were challenged with an i.v. injection via the tail vein of either $0.5 \times 10^6$ or $1 \times 10^6$ cells of the human B-cell acute lymphoblastic leukaemia (ALL) cell line, NALM-6, which has been stably transduced to express a firefly luciferase (FLuc) and green fluorescent protein (GFP) gene. Mice were subsequently administered with or without a single i.v. injection via the tail vein of $20 \times 10^6$ GDX012 cells 24 hours or 6 days after tumour challenge. Control and treated mice all received i.p. injections of recombinant human IL-15 (1 μg/mouse every 2-3 days for the duration of the study) to support GDX012 survival. Tumour burden was assessed twice a week using s.c. administration of luciferin and in-life whole body bioluminescence imaging (BLI). After 4 weeks, mice were terminated and hind limb long bones removed. The bone marrow was flushed from the hind limb long bones using RPMI-1640 and collected for flow cytometric analysis. Briefly, cells were stained with eFluor780 fixable live/dead discrimination dye, then stained with FITC-conjugated anti-human CD45, PE-conjugated anti-human CD19 and APC-conjugated anti-human TCRγδ antibodies. Cells were finally fixed in 4% paraformaldehyde and run on a MACSQUANT10 flow cytometer.

A single administration of GDX012 in a systemic ALL *in vivo* model was able to reduce the growth of disseminated tumour compared with controls (**Figure 12**). In addition, GDX012 cells were able to home to and preferentially control tumour burden specifically within the bone marrow (**Figure 13**).

## CLAUSES

A set of clauses defining the invention and its preferred aspects is as follows:

Clause 1. An allogeneic composition comprising Vδ1+ T cells for use in the treatment of a patient with a myeloid malignancy.

Clause 2. The allogeneic composition for use as defined in clause 1, wherein the myeloid malignancy is selected from acute myeloid leukaemia (AML) and myelodysplastic syndrome (MDS).

Clause 3. The allogeneic composition for use as defined in clause 1 or clause 2, wherein the patient is positive for minimal residual disease (MRD+).

Clause 4. The allogeneic composition for use as defined in clause 3, wherein the MRD+ patient is in complete remission, contains no detectable leukaemic blasts in the peripheral blood and contains less than 5% leukaemic blasts in the bone marrow.

Clause 5. The allogeneic composition for use as defined in any one of clauses 1 to 4, wherein the patient has previously been treated with chemotherapy.

Clause 6. The allogeneic composition for use as defined in clause 5, wherein the patient has been treated with chemotherapy at least 3 days prior to administration of the allogeneic composition.

Clause 7. The allogeneic composition for use as defined in clause 5 or clause 6, wherein the chemotherapy is selected from fludarabine and cyclophosphamide.

Clause 8. The allogeneic composition for use as defined in any one of clauses 1 to 7, which comprises at least about 90% CD45+ cells relative to total live cells.

Clause 9. The allogeneic composition for use as defined in any one of clauses 1 to 8, which comprises at least about 60% γδ T cells relative to total live cells.

Clause 10. The allogeneic composition for use as defined in any one of clauses 1 to 9, which comprises at least about 50% Vδ1+ T cells relative to total live cells.

Clause 11. The allogeneic composition for use as defined in any one of clauses 1 to 10, which comprises less than about $1 \times 10^{10}$ total live cells.

Clause 12. The allogeneic composition for use as defined in clause 11, which comprises less than about $1 \times 10^9$ total live cells.

Clause 13. The allogeneic composition for use as defined in clause 12, which comprises less than about $1 \times 10^8$ total live cells.

Clause 14. The allogeneic composition for use as defined in any one of clauses 1 to 11, wherein the allogeneic composition comprises about $8 \times 10^9$, $4 \times 10^9$, $2.4 \times 10^9$, $1.2 \times 10^9$, $8 \times 10^8$, $4 \times 10^8$, $8 \times 10^7$ or $4 \times 10^7$ total live cells.

Clause 15. A dose comprising the allogeneic composition for use as defined in any one of clauses 1 to 14.

Clause 16. The dose as defined in clause 15, which comprises less than about $1 \times 10^5$ cells/kg.

Clause 17. The dose as defined in clause 15, which comprises less than about $1 \times 10^6$ cells/kg.

Clause 18. The dose as defined in clause 15, which comprises less than about $1 \times 10^7$ cells/kg.

Clause 19. The dose as defined in clause 15, which comprises less than about $3 \times 10^7$ cells/kg.

Clause 20. The dose as defined in clause 15, which comprises less than about $1 \times 10^8$ cells/kg.

Clause 21. The dose as defined in clause 15, which comprises less than about $5 \times 10^4$ $\alpha\beta$ T cells/kg.

Clause 22. The dose as defined in clause 21, which comprises less than about $1 \times 10^4$ $\alpha\beta$ T cells/kg.

Clause 23. The allogeneic composition for use as defined in any one of clauses 1 to 15, wherein the $V\delta1+$ T cells are obtained from a sample by a method comprising culturing the sample in a medium comprising a T cell mitogen and a growth factor having interleukin-4-like activity, in the absence of a growth factor having interleukin-15-like activity.

Clause 24. The allogeneic composition for use as defined in any one of clauses 1 to 15, wherein the $V\delta1+$ T cells are obtained from a sample by a method comprising culturing the sample in a medium comprising a T cell mitogen and a growth factor having interleukin-15-like activity, in the absence of a growth factor having interleukin-4-like activity.

Clause 25. The allogeneic composition for use as defined in clause 23 or clause 24, wherein the $V\delta1+$ T cells are collected after at least 11 days of culturing.

Clause 26. The allogeneic composition for use as defined in any one of clauses 23 to 25, wherein the culturing is performed in a vessel comprising a gas permeable material.

Clause 27. The allogeneic composition for use as defined in clause 26, wherein the vessel comprises a liquid sealed container comprising a gas permeable material to allow gas exchange.

Clause 28. The allogeneic composition for use as defined in clause 26 or clause 27, wherein the bottom of said vessel is configured to allow gas exchange from the bottom of the vessel.

Clause 29. The allogeneic composition for use as defined in any one of clauses 23 to 28, wherein the sample is cultured in serum-free medium.

Clause 30. The allogeneic composition for use as defined in any one of clauses 23 to 28, wherein the sample is cultured in media containing serum or serum-replacement.

Clause 31. A method of treating a myeloid malignancy comprising administering a therapeutically effective amount of an allogeneic composition comprising $V\delta1+$ T cells to a patient with said myeloid malignancy.

Clause 32. The method as defined in clause 31, wherein the myeloid malignancy is selected from AML and MDS.

Clause 33. The method as defined in clause 31 or clause 32, wherein the patient is positive for minimal residual disease (MRD+).

Clause 34. The method as defined in clause 33, wherein the MRD+ patient is in complete remission, contains no detectable leukaemic blasts in the peripheral blood and contains less than 5% leukaemic blasts in the bone marrow.

Clause 35. The method as defined in any one of clauses 31 to 34, which additionally comprises administration of chemotherapy.

Clause 36. The method as defined in clause 35, wherein the patient is treated with chemotherapy at least 3 days prior to administration of the allogeneic composition.

Clause 37. The method as defined in clause 35 or clause 36, wherein the chemotherapy is selected from fludarabine and cyclophosphamide.

Clause 38. The method as defined in clause 31, wherein the therapeutically effective amount comprises about $8 \times 10^9$, $4 \times 10^9$, $2.4 \times 10^1$, $1.2 \times 10^9$, $8 \times 10^8$, $4 \times 10^8$, $8 \times 10^7$ or $4 \times 10^7$ total live cells.

Clause 39. The method as defined in clause 31, wherein the therapeutically effective amount comprises less than about $1 \times 10^{10}$ total live cells.

Clause 40. The method as defined in clause 31, wherein the therapeutically effective amount comprises less than about $1 \times 10^9$ total live cells.

Clause 41. The method as defined in clause 31, wherein the therapeutically effective amount comprises less than about $1 \times 10^8$ total live cells.

Clause 42. The method as defined in clause 31, wherein the therapeutically effective amount comprises less than about $5 \times 10^4$ $\alpha\beta$ T cells/kg.

Clause 43. The dose as defined in clause 42, wherein the therapeutically effective amount comprises less than about $1 \times 10^4$ $\alpha\beta$ T cells/kg.

**Claims**

1. An allogeneic composition comprising V$\delta$1+ T cells for use in the treatment of a patient with a myeloid malignancy, wherein the patient is positive for minimal residual disease (MRD+), wherein the myeloid malignancy comprises acute myeloid leukaemia (AML), and wherein the MRD+ patient is in complete remission, contains no detectable leukaemic blasts in the peripheral blood and contains less than 5% leukaemic blasts in the bone marrow.

2. The allogeneic composition for use as defined in claim 1, wherein the patient has previously been treated with chemotherapy.

3. The allogeneic composition for use as defined in claim 2, wherein the patient has been treated with chemotherapy at least 3 days prior to administration of the allogeneic composition.

4. The allogeneic composition for use as defined in claim 2 or claim 3, wherein the chemotherapy is selected from fludarabine and cyclophosphamide.

5. The allogeneic composition for use as defined in any one of claims 1 to 4, which comprises at least about 90% CD45+ cells relative to total live cells.

6. The allogeneic composition for use as defined in any one of claims 1 to 5, which comprises at least about 60% $\gamma\delta$ T cells relative to total live cells.

7. The allogeneic composition for use as defined in any one of claims 1 to 6, which comprises at least about 50% V$\delta$1+ T cells relative to total live cells.

8. The allogeneic composition for use as defined in any one of claims 1 to 7, which comprises less than about $1 \times 10^{10}$ total live cells.

9. The allogeneic composition for use as defined in claim 8, which comprises less than about $1 \times 10^9$ total live cells.

10. The allogeneic composition for use as defined in claim 9, which comprises less than about $1 \times 10^8$ total live cells.

11. The allogeneic composition for use as defined in any one of claims 1 to 8, wherein the allogeneic composition comprises about $8 \times 10^9$, $4 \times 10^9$, $2.4 \times 10^9$, $1.2 \times 10^9$, $8 \times 10^8$, $4 \times 10^8$, $8 \times 10^7$ or $4 \times 10^7$ total live cells.

12. The allogenic composition for use as defined in any one of claims 1 to 11, which is to be administered at a dose of less than about 5 x $10^4$ $\alpha\beta$ T cells/kg.

13. The allogenic composition for use as defined in claim 12, which is to be administered at a dose of less than about 1 x $10^4$ $\alpha\beta$ T cells/kg.

14. The allogenic composition for use as defined in any one of claims 1 to 13, wherein the V$\delta$1+ T cells are obtained from a sample by a method comprising culturing the sample in a medium comprising a T cell mitogen and a growth factor having interleukin-4-like activity, in the absence of a growth factor having interleukin-15-like activity.

15. The allogenic composition for use as defined in any one of claims 1 to 13, wherein the V$\delta$1+ T cells are obtained from a sample by a method comprising culturing the sample in a medium comprising a T cell mitogen and a growth factor having interleukin-15-like activity, in the absence of a growth factor having interleukin-4-like activity.

FIGURE 1

FIGURE 1 (contd.)

**A**

**B**

FIGURE 2

FIGURE 3

**A**

**B**

FIGURE 4

**C**

**Healthy leukocytes**

**D**

**Ex vivo Vδ1+ cells**

FIGURE 4 (contd.)

**E**

Gated on CD3+ Vδ1+

**F**

FIGURE 4 (contd.)

FIGURE 5

**A**

Primary AML cells
(1x 10$^6$ cells)
i.b.m

VD1 cells or PBS
(2 x 10$^7$ cells)
tail i.v

NSGS

7-10

Tumor burden
(Weekly bleedings)

Survival
assessement

Day 0     + 1     + 8     + 15

Tumor trigger in the blood
(>100 tumor cells/mL)

Sublethal irradiation
(200 – 225 rad)

**B**

KG-1 AML cells
(2 x 10$^6$ cells)
tail i.v

VD1 cells or PBS
(2 x 10$^7$ cells)
tail i.v

NSG

Tumor burden
(Weekly bleedings)

Survival
assessement

Day 0     10     17     24

Sublethal irradiation
(200 – 225 rad)

**C**

HEL AML
(1 x 10$^4$ cells)
i.b.m

VD1 cells or PBS
(2 x 10$^7$ cells)
tail i.v

NRGS

7-10

Survival
assessement

Day 0     + 1     + 8     + 15

Tumor burden
(Weekly bleedings)

Tumor trigger in the blood
(>100 tumor cells/mL)

Sublethal irradiation
(225 – 250 rad)

CTR
VD1

Survival (%)

$p < 0.05$

Days after tumor trigger

FIGURE 6

FIGURE 6 (contd.)

**G**

**H**

**I**

FIGURE 6 (contd.)

FIGURE 7

FIGURE 7 (contd.)

HL-60 (2ng/ml IL-15)

C1 - Tumour cell challenge 1
C2 - Tumour cell challenge 2

FIGURE 8

**A**

FIGURE 9

**B**

FIGURE 9 (contd.)

**C**

FIGURE 9 (contd.)

FIGURE 9 (contd.)

Cytotoxicity (NALM-6 + B cell)

Legend:
- B cell
- B cell + GDX012
- NALM-6
- NALM-6 + GDX012

Y-axis: % Sytox$^+$

X-axis: E:T Ratio (1:1, 2:1, 5:1, 10:1)

FIGURE 10

## αβ T cells vs PBLs

## αβ T cells or GDX012 vs PBLs

*N.D= not detected

FIGURE 11

FIGURE 12

FIGURE 12 (contd.)

GDX012 in the bone marrow: study end point

FIGURE 13

FIGURE 13 (contd.)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2016198480 A
- WO 2017072367 A
- WO 2018202808 A
- WO 2005035728 A
- US 9255243 B

### Non-patent literature cited in the description

- **DENIGER et al.** *Clin. Cancer Res.*, 2014, vol. 20 (22), 5708-5719
- **GENTLES et al.** *Nat. Med.*, 2015, vol. 21 (8), 938-945
- **MURATI et al.** *BMC Cancer*, 2012, vol. 12, 304
- **TEFFERI** ; **VARDIMAN**. *Leukemia*, 2008, vol. 22, 14-22
- **ALMEIDA et al.** *Cancer Res.*, 2016, vol. 22, 5795-804
- **VERSTICHEL et al.** *Sci. Immunol.*, 2017, vol. 2, eaah4232
- **RAVENS et al.** *Nat. Immunol.*, 2017, vol. 18, 393-401
- **DI LORENZO et al.** *Sci Data*, 2019, vol. 6, 115
- **NAIK et al.** *Nature*, 2013, vol. 496, 229-232
- **NOBREGA-PEREIRA et al.** *Cancer Res.*, 2018, vol. 78, 731-741
- **MARDIROS et al.** *Blood*, 2013, vol. 122, 3138-3148
- **GILL et al.** *Blood*, 2014, vol. 123, 2343-2354
- **PETROV et al.** *Leukemia*, 2018, vol. 32, 1317-1326